# FASCICULE DE BREVET EUROPEEN

(11) **EP 1 746 969 B1**
(45) Date de publication et mention de la délivrance du brevet: **11.03.2009**
(21) Numéro de dépôt: 05762561.8
(22) Date de dépôt: 17.05.2005
(51) Int. Cl.: A61K 8/18, A61Q 3/02

(54) **FILM DE VERNIS A ONGLES RETICULÉ**
VERNETZTER NAGELLACKFILM
CROSS-LINKED NAIL VARNISH FILM

(30) Priorité: 19.05.2004 FR 0451005; 29.06.2004 US 583486 P
(43) Date de publication de la demande: 31.01.2007
(73) Titulaire: L'ORÉAL, 75008 Paris (FR)
(72) Inventeur: ILEKTI, Philippe, F-94700 MAISONS ALFORT (FR)
(74) Mandataire: Le Coupanec, Pascale A.M.P.
(86) Numéro de dépôt international: PCT/FR2005/050336
(87) Numéro de publication internationale: WO 2005/112874

(56) Documents cités:
- EP-A- 1 371 693
- WO-A-97/24103
- WO-A-20/06071768
- DE-A1- 4 140 888
- FR-A- 2 803 743
- GB-A- 1 280 631
- US-A- 4 943 462
- US-A- 5 601 808
- US-A- 5 747 018
- US-A- 2004 000 317
- US-B1- 6 277 358
- DATABASE WPI Section Ch, Week 198827 Derwent Publications Ltd., London, GB; Class A14, AN 1988-186214 XP002316198 & JP 63 122771 A (SUNSTAR KK) 26 mai 1988 (1988-05-26)

## Description

La présente invention concerne un article souple destiné à être appliqué sur les ongles pour leur maquillage et/ou leur soin.

Classiquement, le maquillage des ongles ou des faux ongles est réalisé à l'aide de compositions liquides de maquillage, encore communément appelées vernis à ongles. Ce vernis à ongles est appliqué généralement sous la forme de couches superposées à la surface de l'ongle à maquiller, en respectant une étape de séchage intermédiaire entre chaque couche de vernis appliquée. En fait, ce mode de maquillage ne s'avère pas totalement satisfaisant.

Tout d'abord, son application nécessite un certain temps.

Par ailleurs, ce type de maquillage implique d'être répété à bref délai à cause de sa tenue insuffisante. En effet, très rapidement, généralement au terme de trois à cinq jours, le vernis appliqué s'écaille et sa brillance diminue. Il est alors nécessaire de procéder à une étape de démaquillage et de répéter une nouvelle opération de maquillage.

Enfin, les formules de vernis à ongles classiques impliquent généralement la mise en oeuvre de solvants volatils qui génèrent, lors de l'application, une odeur inconfortable.

Plusieurs alternatives ont déjà été proposées pour tenter de surmonter, au moins en partie, les inconvénients précités. Ainsi, des produits de maquillage des ongles ont été proposés sous forme de kit de deux compositions liquides de vernis à ongles. Toutefois, l'amélioration de tenue est, dans ce cas, acquise au détriment des conditions d'application qui multiplient par deux le nombre de couches à appliquer.

Une autre alternative a consisté à développer des compositions de vernis à ongles à base de dispersion de polymères en phase aqueuse, et donc satisfaisantes sur le plan olfactif. Malheureusement, les vernis correspondants s'avèrent présenter une tenue insuffisante dans le temps.

La présente invention vise précisément à proposer un mode de maquillage et/ou de soin des ongles ou des faux ongles qui soit précisément, par opposition aux formulations liquides classiques de type vernis à ongles, facile et aisé en terme d'application, doté d'une tenue dans le temps significativement améliorée et d'une teneur nettement réduite en solvant(s) organique(s).

Plus précisément, la présente invention concerne selon un premier de ses aspects, un article souple destiné à être appliqué sur des ongles et/ou faux ongles pour leur maquillage et/ou leur soin comportant :
- au moins une couche adhésive permettant la fixation de l'article sur l'ongle et
- au moins un film réticulé, notamment coloré ou transparent, comme décrit à la revendication 1 et la revendication 2.

Dans une variante, l'article peut comprendre une superposition d'au moins deux voire plus de films réticulés différents.

En particulier, l'un d'entre eux, généralement celui en contact avec la couche adhésive, peut être coloré et l'autre transparent.

Selon une autre variante de l'invention, l'article peut comprendre en outre entre la couche adhésive et le film réticulé au moins un film de vernis coloré. Un tel film peut dériver de l'évaporation de la phase solvant organique ou aqueuse d'une solution ou dispersion, généralement colorée, d'au moins un polymère filmogène. Ce polymère filmogène peut notamment être choisi parmi la nitrocellulose ou des esters de cellulose.

Selon cette variante de l'invention, le film réticulé est de préférence transparent.

Tel qu'utilisé ici, le terme « transparent » signifie que le revêtement réticulé a un index HAZEBYK de moins de 5 tel que mesuré avec un brillancemètre de type KYKHAZEGLOSS.

Selon un deuxième aspect, la présente invention concerne un procédé de préparation d'un article souple de maquillage et/ou de soin des ongles comprenant au moins les étapes consistant à superposer sur un support amovible :
a) au moins une couche d'une composition à base d'au moins un matériau adhésif, et
b) au moins une couche d'une composition réticulable, la réticulation de ladite composition étant réalisée consécutivement au dépôt de celle-ci de manière à obtenir un film réticulé.

Selon une première variante de l'invention le procédé comprend au moins les étapes consistant à :
a) déposer sur un support amovible au moins une couche d'une composition contenant au moins un matériau adhésif,
b) déposer sur ladite couche adhésive au moins une couche d'une composition réticulable,
c) réticuler ladite composition de manière à obtenir un film réticulé, et
d) si nécessaire sécher au moins partiellement ledit article.

Selon une seconde variante de l'invention, le procédé comprend au moins les étapes consistant à :
a) déposer sur un support amovible au moins une couche d'une composition réticulable,
b) réticuler ladite composition de manière à obtenir un film réticulé,
c) si nécessaire sécher au moins partiellement ledit film,
d) déposer sur ledit film obtenu en c) au moins une couche d'une composition contenant au moins un matériau adhésif,
e) si nécessaire sécher au moins partiellement ledit article,
f) recouvrir la couche adhésive obtenue en e) par un support amovible, et, le cas échéant,
g) récupérer ledit article par pelage du film réticulé du support en a).

Selon une autre variante de l'invention, ledit procédé comprend en outre au moins une étape consistant à former entre la couche adhésive et le film réticulé, un film de vernis coloré.

Selon un troisième aspect, la présente invention concerne un produit de maquillage et/ou de soin des ongles et/ou des faux ongles comprenant dans un conditionnement, sensiblement étanche à l'air, au moins un article conforme à l'invention, le conditionnement étant tel que l'article s'y trouve conservé sous une forme partiellement sèche.

Au sens de la présente invention, le terme partiellement sec entend qualifier le fait que l'article obtenu après formation du film réticulé n'est pas totalement exempt du solvant résiduel. En particulier, il possède une teneur en matière sèche inférieure à 80 %, en particulier inférieure à 75 % et plus particulièrement inférieure à 70 % en poids par rapport à son poids total.

Selon un mode de réalisation particulier, ce conditionnement comporte un réservoir, comme par exemple une poche, souple ou non, apte à contenir de manière étanche un produit pour préserver ledit article d'un séchage total et prématuré avant son utilisation. Plus précisément, le conditionnement est étanche à l'air et/ou aux solvants.

Selon un quatrième aspect, la présente invention concerne un procédé de préparation d'un produit tel que défini ci-dessus comprenant les étapes consistant à superposer sur un support amovible :
- au moins une couche d'une composition à base d'au moins un matériau adhésif,
- au moins une couche d'une composition réticulable, la réticulation étant réalisée consécutivement au dépôt de ladite composition,
- si nécessaire le séchage partiel dudit article ainsi obtenu, et
- le conditionnement dudit article à un état partiellement sec au sein d'un conditionnement sensiblement étanche à l'air.
   Selon ce mode de réalisation, l'article n'acquiert un aspect totalement sec, et donc sa forme définitive qu'après application sur l'ongle, par simple exposition à l'air ambiant.
   Selon un cinquième aspect, la présente invention concerne un procédé de maquillage et/ou de soin des ongles comprenant le fait d'appliquer sur un ongle la face adhésive d'un article selon l'invention.
   D'une manière générale, l'article selon la présente invention se présente sous la forme d'un film.
   Au sens de la présente invention, le terme « souple » qualifie une flexibilité suffisante de ce film, c'est-à-dire propice à des déformations mécaniques de type étirement pour l'ajuster à la surface d'un ongle. Cette déformabilité est notamment caractérisée par le paramètre de déformation à la rupture εᵣ discuté ci-après.
   L'article selon l'invention se différencie notamment à ce titre d'un article de type faux ongle qui se caractérise par une rigidité incompatible avec une telle déformation mécanique.
   Une autre différence entre l'article conforme à l'invention et un faux ongle, réside dans la sensibilité de cet article vis-à-vis des solvants organiques polaires du type acétone, ester et/ou alcool court. En effet, le film réticulé figurant sur la face externe de l'article selon l'invention, c'est-à-dire non adhérente à l'ongle, possède une aptitude à augmenter de volume et donc de poids lorsqu'il est mis en contact avec l'un de ces solvants. Un faux ongle est totalement dénué d'une telle sensibilité. Cette aptitude à gonfler, manifestée par l'article selon l'invention, est précisément avantageuse pour son élimination lorsque celui-ci est appliqué en surface d'un ongle ou d'un faux ongle. En effet, l'article selon l'invention peut être aisément éliminé par simple démaquillage à l'aide d'un dissolvant classique, par opposition à un faux ongle qui se retire. Ainsi, l'article selon l'invention est avantageusement démaquillable par des solvants organiques et notamment par les acétates d'alkyles et leurs mélanges.
   L'article conforme à l'invention présente en outre une tenue dans le temps significative et notamment à l'échelle d'au moins une semaine. Il s'avère ainsi résistant à l'eau, aux frottements et aux chocs, et ne présente pas d'usure ni d'écaillage significatifs dans ce délai.
   L'article selon l'invention peut être utilisé soit à des fins de maquillage, auquel cas il comprend généralement au moins un film réticulé coloré ou à des fins de protection vis-à-vis d'un film de vernis. Dans cette alternative, il comprend généralement un film réticulé transparent.

### I. ARTICLE SELON L'INVENTION

L'article selon l'invention peut se caractériser par un extrait sec élevé. En effet, la quantité de matière sèche est supérieure à 80%, et en particulier supérieure à 85 % et en particulier supérieur à 90 % en poids par rapport au poids total de l'article. Autrement dit la quantité de solvant volatil est inférieure à 20 %, en particulier inférieure à 15 % et plus particulièrement inférieure à 10 % en poids par rapport au poids total de l'article.

Toutefois, selon un autre mode de réalisation privilégié, l'article selon l'invention peut avantageusement se présenter sous une forme partiellement sèche. Dans ce cas particulier, l'article est conditionné dans un réservoir comme par exemple une poche, souple ou non, suffisamment étanche pour lui préserver cet aspect partiellement sec. Ce n'est qu'au moment de son utilisation et par conséquent lors de sa mise en contact avec l'air, que l'article sèche totalement pour acquérir la teneur en matière sèche décrite précédemment.

Dans un produit selon l'invention, l'article selon l'invention possède avantageusement une teneur en matière sèche inférieure à 80 %, notamment inférieure à 75 % et plus particulièrement inférieure à 70 % en poids par rapport à son poids total. Ledit article peut par ailleurs posséder une teneur en matière sèche supérieure à 60 %, notamment supérieure à 65 % en poids par rapport à son poids total. Un tel article, lorsqu'il est extrait du conditionnement d'un produit conforme à l'invention et exposé à l'air ambiant, acquiert un état sec tel que défini ci-dessus au terme de 24 h.

De préférence, la quantité de matière sèche, communément appelée « extrait sec » des articles selon l'invention, est mesurée par échauffement de l'échantillon par des rayons infrarouges de 2 µm à 3,5 µm de longueur d'onde. Les substances contenues dans lesdits films qui possèdent une pression de vapeur élevée, s'évaporent sous l'effet de ce rayonnement. La mesure de la perte de poids de l'échantillon permet de déterminer « l'extrait sec » de l'article. Ces mesures sont réalisées au moyen d'un dessiccateur à infrarouges commercial LP16 de chez Mettler. Cette technique est parfaitement décrite dans la documentation de l'appareil fournie par Mettler.

Le protocole de mesure est le suivant.

On dépose environ 10 g d'échantillon d'un article sur une coupelle métallique. Celle-ci, après introduction dans le dessiccateur, est soumise à une consigne de température de 120 °C pendant une heure. La masse humide de l'échantillon, correspondant à la masse initiale et la masse sèche de l'échantillon, correspondant à la masse après exposition au rayonnement, sont mesurées au moyen d'une balance de précision.

La teneur en matière sèche est calculée de la manière suivante : $Extrait Sec = 100 \times ( masse sèche / masse humide ) .$

### Reprise à l'eau

L'article selon l'invention peut être caractérisé à l'état sec par une reprise à l'eau portée à 25 °C inférieure ou égale à 20 %, notamment inférieure ou égale à 16 %, et en particulier inférieure à 10 %.

Selon la présente demande, on entend par "reprise à l'eau", le pourcentage d'eau absorbé par l'article après 60 minutes d'immersion dans l'eau, à 25 °C (température ambiante). La reprise à l'eau est mesurée pour des morceaux d'environ 1 cm² découpés dans l'article sec. Ils sont pesés (mesure de la masse M1) puis immergés dans l'eau pendant 60 minutes ; après immersion, le morceau de film est essuyé pour éliminer l'excédent d'eau en surface puis pesé (mesure de la masse M2). La différence M2 - M1 correspond à la quantité d'eau absorbée par le film.

La reprise à l'eau est égale à [(M2 - M1) / M1] x 100 et est exprimée en pourcentage de poids par rapport au poids du film.

### Module de conservation E'

Par ailleurs, l'article selon l'invention est avantageusement un film ayant un module de conservation E' supérieur ou égal à 1 MPa, notamment allant de 1 MPa à 5000 MPa, en particulier supérieur ou égal à 5 MPa, notamment allant de 5 à 1000 MPa, et plus particulièrement supérieur ou égal à 10 MPa par exemple allant de 10 à 500 MPa à une température de 30 °C et une fréquence de 0,1 Hz.

La mesure du module de conservation est effectuée par DMTA (Dynamical and Mechanical Temperature Analysis ou Analyse dynamique et mécanique en température).

On effectue des essais de viscoélasticimétrie avec un appareil DMTA de Polymer TA Instruments (modèle DMA2980), sur un échantillon d'article. On découpe (par exemple à l'emporte-pièce) les éprouvettes. Celles-ci ont typiquement une épaisseur d'environ 150 µm, une largeur de 5 à 10 mm et une longueur utile d'environ 10 à 15 mm.

Les mesures sont effectuées à une température constante de 30°C.

L'échantillon est sollicité en traction et en petites déformations (on lui impose par exemple un déplacement sinusoïdal de ± 8 µm) lors d'un balayage en fréquence, la fréquence allant de 0,1 à 20 Hz. On travaille ainsi dans le domaine linéaire, sous de faibles niveaux de déformation.

Ces mesures permettent de déterminer le module complexe E* = E' + iE" du film de composition testé, E' étant le module de conservation et E" le module dit de perte.

### Déformation et/ou Energie à la rupture

Avantageusement, les articles selon l'invention, possèdent une déformation à la rupture εᵣ supérieure ou égale à 5 %, notamment allant de 5 à 500 %, de préférence supérieure ou égale à 15 %, notamment allant de 15 à 400 % et/ou une énergie à rupture par unité de volume Wᵣ supérieure ou égale à 0,2 J/cm³, notamment allant de 0,2 à 100 J/cm³, de préférence supérieure à 1 J/cm³, notamment allant de 1 à 50 J/cm³.

La déformation à la rupture et l'énergie à rupture par unité de volume sont déterminées par des essais de traction effectués sur un film réticulé d'environ 200 µm d'épaisseur.

Pour effectuer ces essais, l'article est découpé en éprouvettes haltères de longueur utile 33 ± 1 mm et de largeur utile 6 mm. La section (S) de l'éprouvette est alors définie comme : S = largeur x épaisseur (cm²) ; cette section sera utilisée pour le calcul de la contrainte.

Les essais sont réalisés, par exemple, sur un appareil de traction commercialisé sous l'appellation Lloyd^{®} LR5K. Les mesures sont réalisées à température ambiante (20 °C).

Les éprouvettes sont étirées à une vitesse de déplacement de 33 mm/min, correspondant à une vitesse de 100 % d'allongement par minute.

On impose donc une vitesse de déplacement et on mesure simultanément l'allongement ΔL de l'éprouvette et la force F nécessaire pour imposer cet allongement. C'est à partir de ces données ΔL et F que l'on détermine les paramètres contraintes σ et déformation ε.

Il est ainsi obtenu une courbe contrainte σ = (F/S) en fonction de la déformation ε = (ΔL/Lₒ) x 100, l'essai étant conduit jusqu'à rupture de l'éprouvette, Lₒ étant la longueur initiale de l'éprouvette.

La déformation à la rupture εᵣ est la déformation maximale de l'échantillon avant le point de rupture (en %).

L'énergie à rupture par unité de volume Wᵣ en J/cm³ est définie comme la surface sous cette courbe contrainte/déformation telle que : $Wr = {\int }_{0}^{{ε}_{r}} σ . ε . ⅆ ε$

### FILM RETICULE

Comme précisé précédemment, le film selon l'invention dérive d'une réticulation.

Au sens de la présente invention, un film qualifié de réticulé peut être totalement ou partiellement réticulé.

Dans le cas d'une réticulation partielle, celle-ci est bien entendu suffisante pour former le film attendu.

Cette réticulation peut ainsi être réalisée par voie thermique, photochimique et/ou chimique, en présence ou non d'un catalyseur. La réalisation de cette réticulation relève des compétences de l'homme de l'art.

Bien entendu, les composés mis en présence pour la réticulation sont choisis, notamment selon la nature des fonctions réactives qu'ils possèdent respectivement, pour être capable d'interagir dans les conditions de la réaction de réticulation considérée.
- Selon une première variante, la réaction de réticulation s'apparente à une réaction de polyaddition ou polycondensation réalisée en présence ou en absence de catalyseur.

Dans ce cas particulier, la composition réticulable contient un au moins un système réactif formé par :
- au moins un premier composé (A) comprenant au moins deux fonctions X, et
- au moins un deuxième composé (B) comprenant au moins deux fonctions Y, réactives vis-à-vis des fonctions X.

Avantageusement, le système réactif possède une fonctionnalité moyenne (nombre total des fonctions X et Y/nombre total des molécules de composés (A) et (B)) supérieure à 2 de manière à procurer un réseau tridimensionnel.

Plus particulièrement, pour l'obtention d'un effet de réticulation satisfaisant, la fonctionnalité moyenne du système réactif peut être au moins égale à 2,2 et plus particulièrement varier de 2,5 à 100.

Les composés (A) et (B) peuvent être d'origine organique et notamment de type oligomère, polymère et/ou copolymère ou de nature inorganique à l'image par exemple d'une particule minérale, en quel cas ils possèdent en surface les deux fonctions X ou Y requises.

Les fonctions X et Y réactives les unes vis-à-vis des autres sont choisies parmi des fonctions dites réactives et des fonctions comportant au moins un hydrogène labile.

Plus précisément, les fonctions réactives sont choisies parmi les fonctions isocyanates, époxydes et les doubles liaisons éthyléniques et les fonctions à hydrogène(s) labile(s) sont du type carboxylique, alcool notamment phénolique, amine primaire ou secondaire, amide, aminoalcool et/ou thiol.

Plus particulièrement, les composés (A) et (B) mis en présence, possèdent respectivement au moins deux fonctions dites réactives et notamment de type époxyde et/ou isocyanate et au moins deux fonctions à hydrogène(s) labile(s) notamment de type amine ou aminoalcool et peuvent notamment être choisis parmi les composés cités précédemment.

Par exemple, X peut être une fonction époxyde et/ou isocyanate et Y peut être choisi parmi une fonction acide carboxylique et/ou une fonction anhydride et/ou une fonction amine et/ou une fonction thiol et/ou une fonction hydroxyle, en particulier phénolique.

### Les composés à fonctions isocyanates :

Les composés comportant au moins deux fonctions isocyanate libres sont connus dans la technique. Il peut s'agir de polyisocyanates, y compris des diisocyanates ou triisocyanates, pouvant avoir une masse moléculaire inférieure à 500 000, notamment inférieure à 10 000. Ces polyisocyanates sont généralement obtenus par polyaddition, polycondensation et/ou greffage, portant au moins deux fonctions isocyanates, soit aux extrémités de chaîne soit sur des groupes latéraux.

Les polyisocyanates peuvent être linéaires, ramifiés, aliphatiques, cycloaliphatiques ou aromatiques.

Parmi les composés de ce type utilisables, on peut citer :
a) Les diisocyanates comportant de 4 à 50 atomes de carbone, notamment de 4 à 30, tels que le 1,4-tétraméthylène diisocyanate, le 1,6-hexaméthylènediisocyanate, le 2,6- et le 2,4-toluènedüsocyanate, le diphénylméthanediisocyanate, et l'isophoronediisocyanate,
b) les triisocyanates de formule : 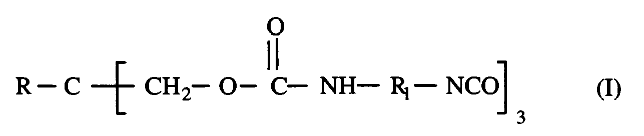 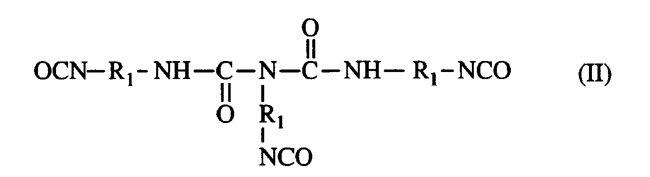 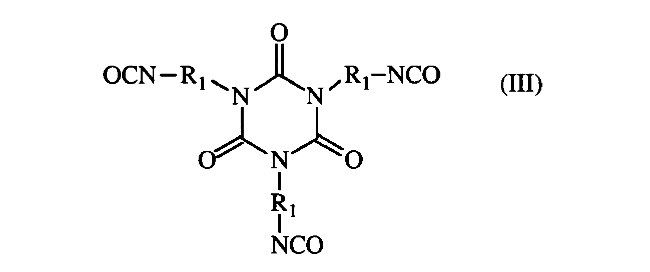 où R est un radical alkyle comportant de 1 à 30 atomes de carbone, chaque R₁ représentant indépendamment un radical divalent hydrocarboné linéaire, ramifié ou cyclique ayant de 2 à 30 atomes de carbone,
c) les polycondensats à groupes isocyanate terminaux ou latéraux notamment tels que les polyuréthannes et/ou polyurées (y compris les copolymères séquencés comportant au moins une séquence polyuréthanne et/ou polyurée et au moins une séquence polyéther, polyester, polysiloxane, alkyde ou polyacrylate), ainsi que les polyesters, polyamides, polyépoxy, polyéthers, perfluoropolyéthers, et
d) les polymères résultant de la copolymérisation de monomères vinyliques, allyliques et/ou (méth)acryliques et de comonomères à insaturation éthylénique comportant une fonction isocyanate libre comme le méthacrylate de 2-isocyanato éthyle.

Comme polyisocyanate, on peut en particulier utiliser le DESMODUR^{®} N de la société BAYER, le TOLONATE^{®} HDB-LV de la société RHODIA.

### Les composés à fonctions époxydes :

Les composés comportant au moins deux fonctions époxydes sont également connus de l'état de la technique. Ils peuvent être de toute nature chimique. Il peut s'agir de diépoxydes ou de polyépoxydes de faibles masses (inférieures ou égales à 5000), ou bien d'oligomères ou de polymères de toute nature chimique, obtenus par polyaddition, polycondensation et/ou greffage, portant au moins deux fonctions époxydes libres, soit aux extrémités de chaînes, soit en groupes latéraux.

On peut citer à titre d'exemples de tels composés :
a) le diglycidyl éther de bisphénol A résultant de la condensation entre le bisphénol A et l'épichlorhydrine, de structure 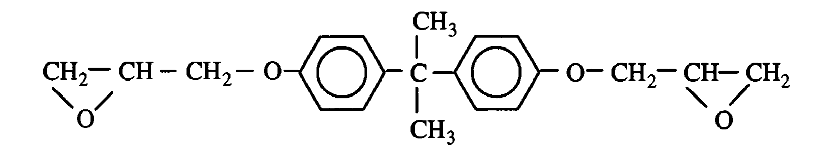
b) les résines diépoxy notamment résultant de la condensation supérieure entre l'éther diglycidique de bisphénol A et l'épichlorhydrine,
c) les résines époxyester à extrémités α,ω-diépoxy notamment résultant de la condensation d'un diacide carboxylique ayant notamment de 2 à 60 atomes de carbone avec un excès stoechiométrique des composés a) ou b),
d) les résines époxyéthers à extrémités α,ω-diépoxy notamment résultant de la condensation d'un diol ayant notamment de 2 à 60 atomes de carbone avec un excès stoechiométrique des composés a) ou b),
e) les huiles naturelles ou synthétiques portant au moins deux groupes époxydes, comme par exemple l'huile de soja époxydée, l'huile de lin époxydée, l'huile de vernonia, notamment décrites dans la demande EP-A-645134,
f) les oligomères ou polymères résultant de la copolymérisation de monomères insaturés ou vinyliques, allyliques et/ou (méth)acryliques, et de comonomères à insaturation éthylénique comportant une fonction époxyde libre (comme le méthacrylate de glycidyle), et
g) les autres polycondensats à groupes époxy terminaux et/ou latéraux tels que les polyesters, polyesteramides, polyamides, alkydes, polyuréthanes et/ou polyurées, polyéthers et perfluoroplyéthers ou silicones.

Des polymères à fonctions époxy sont commercialisés sous les dénominations CYRACURE^{®} UVR-6110, CYRACURE^{®} UVR-6105 , CYRACURE^{®} ERL-4221E, CYRACURE^{®} ERL-4206, CYRACURE^{®} UVR 6128, CYRACURE^{®} UVR 6216 par la société UNION CARBIDE, DER^{®} 439 par la société DOW CHEMICAL, les EPIKATES^{®} 828,1001,1004,1007 de la société SHELL, l'ARALDITE^{®} ECN1299 de la société CIBA-GEIGY, les EPOXYNOVOLACS^{®} de la société DOW CHEMICAL.

### Les composés à doubles liaisons éthyléniques :

Les composés portant des doubles liaisons éthyléniques peuvent être de toute nature chimique. Ils peuvent notamment être choisis parmi :
a) les polyesters à insaturation(s) éthylénique(s).
   Il s'agit d'un groupe de polymères de type polyester présentant une ou plusieurs doubles liaisons éthyléniques, réparties de manière aléatoire dans la chaîne principale du polymère.
   Ces polyesters insaturés sont obtenus par polycondensation d'un mélange :
   - de diacides carboxyliques aliphatiques linéaires ou ramifiés ou cycloaliphatiques comportant notamment de 3 à 50 atomes de carbone, de préférence de 3 à 20 atomes de carbone, tels que l'acide adipique ou l'acide sébacique, de diacides carboxyliques aromatiques ayant notamment de 8 à 50 atomes de carbone, de préférence de 8 à 20 atomes de carbone, tels que les acides phtaliques, notamment l'acide téréphtalique, et/ou de diacides carboxyliques issus de dimères d'acides gras à insaturations éthyléniques tels que les dimères des acides oléique ou linoléique décrits dans la demande EP-A-959 066 (paragraphe [0021]) commercialisés sous les dénominations Pripol^{®} par la société Unichema ou Empol^{®} par la société Henkel, tous ces diacides devant être exempts de doubles liaisons éthyléniques polymérisables,
   - de diols aliphatiques linéaires ou ramifiés ou cycloaliphatiques comportant notamment de 2 à 50 atomes de carbone, de préférence de 2 à 20 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 atomes de carbone tel que le bisphénol A, le bisphénol B, et/ou de dimères diols issus de la réduction des dimères d'acides gras tels que définis précédemment, et
   - d'un ou de plusieurs diacides carboxyliques ou leurs anhydrides comportant au moins une double liaison éthylénique polymérisable et ayant de 3 à 50 atomes de carbone, de préférence de 3 à 20 atomes de carbone, tels que l'acide maléique, l'acide fumarique ou l'acide itaconique.
b) les polyesters à groupes (méth)acrylate latéraux et/ou terminaux :
   Il s'agit d'un groupe de polymères de type polyester obtenus par polycondensation d'un mélange :
   - de diacides carboxyliques aliphatiques linéaires ou ramifiés ou cycloaliphatiques comportant notamment de 3 à 50 atomes de carbone, de préférence de 3 à 20 atomes de carbone, tels que l'acide adipique ou l'acide sébacique, de diacides carboxyliques aromatiques ayant notamment de 8 à 50 atomes de carbone, de préférence de 8 à 20 atomes de carbone, tels que les acides phtaliques, notamment l'acide téréphtalique, et/ou de diacides carboxyliques issus de dimères d'acides gras à insaturation éthyléniques tels que les dimères des acides oléique ou linoléique décrits dans la demande EP-A-959 066 (paragraphe [0021]) commercialisés sous les dénominations Pripol^{®} par la société Unichema ou Empol^{®} par la société Henkel, tous ces diacides devant être exempts de doubles liaisons éthyléniques polymérisables,
   - de diols aliphatiques linéaires ou ramifiés ou cycloaliphatiques comportant notamment de 2 à 50 atomes de carbone, de préférence de 2 à 20 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 atomes de carbone tel que le bisphénol A, le bisphénol B, et
   - d'au moins un monoester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.
      Ces polyesters différent de ceux décrits ci-dessus sous le point a) par le fait que les doubles liaisons éthyléniques ne sont pas situées dans la chaîne principale mais sur des groupes latéraux ou à l'extrémité des chaînes. Ces doubles liaisons éthyléniques sont celles des groupes (méth)acrylate présents dans le polymère.
      De tels polyesters sont commercialisés par exemple par la société UCB sous les dénominations EBECRYL^{®} (EBECRYL^{®} 450 : masse molaire 1600, en moyenne 6 fonctions acrylate par molécule, EBECRYL^{®} 652 : masse molaire 1500, en moyenne 6 fonctions acrylate par molécule, EBECRYL^{®} 800 : masse molaire 780, en moyenne 4 fonctions acrylate par molécule, EBECRYL^{®} 810 : masse molaire 1000, en moyenne 4 fonctions acrylate par molécule, EBECRYL^{®} 50 000 : masse molaire 1500, en moyenne 6 fonctions acrylate par molécule).
c) les polyuréthannes et/ou polyurées à groupes (méth)acrylate notamment obtenus par polycondensation :
   - de diisocyanates, triisocyanates et/ou polyisocyanates aliphatiques cycloaliphatiques et/ou aromatiques ayant notamment de 4 à 50, de préférence de 4 à 30 atomes de carbone, tels que l'hexaméthylènediisocyanate, l'isophoronediisocyanate, le toluènediisocyanate, le diphénylméthanediisocyanate ou les isocyanurates de formule : 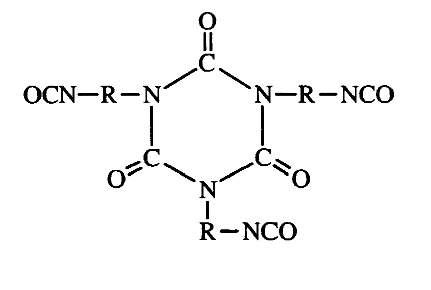 résultant de la trimérisation de 3 molécules de diisocyanates OCN-R-CNO, où R est un radical hydrocarboné linéaire, ramifié ou cyclique comportant de 2 à 30 atomes de carbone ;
   - de polyols, notamment de diols, exempts d'insaturations éthyléniques polymérisables, tels que le 1,4-butanediol, l'éthylèneglycol ou le triméthylolpropane, et/ou de polyamines, notamment de diamines, aliphatiques, cycloaliphatiques et/ou aromatiques ayant notamment de 3 à 50 atomes de carbone, telles que l'éthylènediamine ou l'hexaméthylènediamine, et
   - d'au moins un monoester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.

   De tels polyuréthannes/polyurées à groupes acrylates sont commercialisés par exemple sous la dénomination SR 368 (tris(2-hydroxyéthyl)isocyanurate-triacrylate) ou CRAYNOR^{®} 435 par la société CRAY VALLEY, ou sous la dénomination EBECRYL^{®} par la société UCB (EBECRYL^{®} 210 : masse molaire 1500, 2 fonctions acrylate par molécule, EBECRYL^{®} 230 : masse molaire 5000, 2 fonctions acrylate par molécule, EBECRYL^{®} 270 : masse molaire 1500, 2 fonctions acrylate par molécule, EBECRYL^{®} 8402 : masse molaire 1000, 2 fonctions acrylate par molécule, EBECRYL^{®} 8804 : masse molaire 1300, 2 fonctions acrylate par molécule, EBECRYL^{®} 220 : masse molaire 1000, 6 fonctions acrylate par molécule, EBECRYL^{®} 2220 : masse molaire 1200, 6 fonctions acrylate par molécule, EBECRYL^{®} 1290 : masse molaire 1000, 6 fonctions acrylate par molécule, EBECRYL^{®} 800 : masse molaire 800, 6 fonctions acrylate par molécule).
   On peut également citer les polyuréthannes aliphatiques diacrylate hydrosolubles commercialisés sous les dénominations EBECRYL^{®} 2000, EBECRYL^{®} 2001 et EBECRYL^{®} 2002, et les polyuréthannes diacrylate en dispersion aqueuse commercialisés sous les dénominations commerciales IRR^{®} 390, IRR^{®} 400, IRR^{®} 422 IRR^{®} 424 par la société UCB.
d) les polyéthers à groupes (méth)acrylate obtenus par estérification, par l'acide (méth)acrylique, des groupes hydroxyle terminaux d'homopolymères ou de copolymères d'alkylèneglycols en C₁₋₄, tels que le polyéthylèneglycol, le polypropylèneglycol, les copolymères d'oxyde d'éthylène et d'oxyde de propylène ayant de préférence une masse moléculaire moyenne en poids inférieure à 10 000, le triméthylolpropane polyéthoxylé ou polypropoxylé.
   Des polyoxyéthylènes-di(méth)acrylate de masse molaire appropriée sont commercialisés par exemple sous les dénominations SR 259, SR 344, SR 610, SR 210, SR 603 et SR 252 par la société CRAY VALLEY ou sous la dénomination EBECRYL^{®} 11 par UCB. Des triacrylates de triméthylolpropane polyéthoxylé sont commercialisés par exemple sous les dénominations SR 454, SR 498, SR 502, SR 9035, SR 415 par la société CRAY VALLEY ou sous la dénomination EBECRYL^{®} 160 par la société UCB. Des triacrylates de triméthylolpropane polypropoxylé sont commercialisés par exemple sous les dénominations SR 492 et SR 501 par la société CRAY VALLEY.
e) les époxyacrylates obtenus par réaction entre :
   - au moins un diépoxyde choisi par exemple parmi :
      1) l'éther diglycidylique de bisphénol A,
      2) une résine diépoxy résultant de la réaction entre l'éther diglycidylique de bisphénol A et l'épichlorhydrine,
      3) une résine époxyester à extrémités α,ω-diépoxy résultant de la condensation d'un diacide carboxylique ayant de 3 à 50 atomes de carbone avec un excès stoechiométrique de 1) et/ou 2), et
      4) une résine époxyéther à extrémités α,ω-diépoxy résultant de la condensation d'un diol ayant de 3 à 50 atomes de carbone avec un excès stoechiométrique de 1) et/ou 2),
      5) les huiles naturelles ou synthétiques portant au moins 2 groupes époxyde, telles que l'huile de soja époxydée, l'huile de lin époxydée et l'huile de vernonia époxydée,
      6) un polycondensat phénol-formaldéhyde (résine Novolac^{®}), dont les extrémités et/ou les groupes latéraux ont été époxydés,
         et
   - un ou plusieurs acides carboxyliques ou polyacides carboxyliques comportant au moins une double liaison éthylénique en alpha du groupe carboxylique comme l'acide (méth)acrylique, l'acide crotonique ou les monoesters d'acide (méth)acrylique et d'un diol ou polyol ayant de 2 à 20 atomes de carbone, de préférence de 2 à 6 atomes de carbone tels que le (méth)acrylate de 2-hydroxyéthyle.
      De tels polymères sont commercialisés par exemple sous les dénominations SR 349, SR 601, CD 541, SR 602, SR 9036, SR 348, CD 540, SR 480, CD 9038 par le société CRAY VALLEY, sous les dénominations EBECRYL^{®} 600 et EBECRYL^{®} 609, EBECRYL^{®} 150, EBECRYL^{®} 860, EBECRYL^{®} 3702 par la société UCB et sous les dénominations PHOTOMER^{®} 3005 et PHOTOMER^{®} 3082 par la société HENKEL.
f) les poly(méth)acrylates d'(alkyle en C₁₋₅₀) comportant au moins deux fonctions à double liaison éthylénique portées par les chaînes hydrocarbonées latérales et/ou terminales.
   De tels copolymères sont commercialisés par exemple sous les dénominations IRR^{®} 375, OTA^{®} 480 et EBECRYL^{®} 2047 par la société UCB.
g) les polyorganosiloxanes à groupes (méth)acrylate ou (méth)acrylamide notamment obtenus respectivement
   - par estérification, par exemple d'acide (méth)acrylique et de polyorganosiloxanes, notamment de polydiméthylsiloxanes (PDMS), portant des groupes hydroxyle terminaux et/ou latéraux,
   - par amidification, par exemple d'acide (méth)acrylique et de polyorganosiloxanes porteurs de groupes amine primaire ou secondaire latéraux et/ou terminaux.
      Parmi les PDMS hydroxylés on peut citer les PDMS comportant au moins deux groupes hydroxyalkyle en C₁₋₆ et les diméthicone-copolyols avec des groupes hydroxyle latéraux ou terminaux.
      Des polydiméthylsiloxanes α,ω-dihydroxylés estérifiables sont commercialisés sous les dénominations TEGOMER^{®} H-Si 2111 et TEGOMER^{®} H-Si 2311 par la société GOLDSCHMIDT. Des polydiméthylsiloxanes α,ω-diacrylate sont disponibles à la société SHIN-ETSU sous les références X-22-164 B et X-22-164C.
      Comme PDMS aminés, on peut citer en particulier des PDMS comportant au moins 2 groupes aminoalkyle en C₁₋₁₀, par exemple la silicone aminée commercialisée sous la dénomination Q2-8220 par la société DOW CORNING.
      Avantageusement, les polymères siliconés de ce groupe sont utilisés en mélange avec un ou plusieurs polymères des autres groupes a) à f) décrits ci-dessus, notamment pour modifier le caractère hydrophobe de la composition finale.
h) les perfluoropolyéthers à groupes acrylate notamment obtenus par estérification, par exemple par l'acide (méth)acrylique, de perfluoropolyéthers portant des groupes hydroxyle latéraux et/ou terminaux.
   De tels perfluoropolyéthers α,ω-diols sont décrits notamment dans EP-A-1057849 et sont commercialisés par la société AUSIMONT sous la dénomination FOMBLIN^{®} Z DIOL.
i) les dendrimères et polymères hyperramifiés portant des groupes terminaux (méth)acrylate ou (méth)acrylamide notamment obtenus respectivement par estérification ou amidification de dendrimères et de polymères hyperramifiés à fonctions terminales hydroxyle ou amino, par de l'acide (méth)acrylique.

Les dendrimères (du grec *dendron* = arbre) sont des molécules polymères "arborescentes", c'est-à-dire très ramifiées inventées par D. A. Tomalia et son équipe au début des années 90 (Donald A. Tomalia et al., Angewandte Chemie, Int. Engl. Ed., vol. 29, n° 2, pages 138 - 175). Il s'agit de structures construites autour d'un motif central généralement polyvalent. Autour de ce motif central, sont enchaînés, selon une structure parfaitement déterminée, des motifs ramifiés d'allongement de chaîne donnant ainsi naissance à des macromolécules symétriques, monodispersées ayant une structure chimique et stéréochimique bien définie. Des dendrimères de type polyamidoamine sont commercialisés par exemple sous la dénomination STARBUST^{®} par la société DENDRITECH.

Les polymères hyperramifiés sont des polycondensats, généralement de type polyester, polyamide ou polyéthylèneamine, obtenus à partir de monomères multifonctionnels, qui ont une structure arborescente similaire à celle des dendrimères mais beaucoup moins régulière que celle-ci (voir par exemple WO-A-93/17060 et WO 96/12754).

La société PERSTORP commercialise sous la dénomination BOLTORN^{®} des polyesters hyperramifiés. On trouvera sous la dénomination COMBURST^{®} de la société DENDRITECH des polyéthylèneamines hyperramifiées. Des poly(esteramide) hyperramifiés à extrémités hydroxyle sont commercialisés par la société DSM sous la dénomination HYBRANE^{®}.

Ces dendrimères et polymères hyperramifiés estérifiés ou amidifiés par l'acide acrylique et/ou méthacrylique se distinguent des polymères décrits sous les points a) à h) ci-dessus par le très grand nombre de doubles liaisons éthyléniques présentes.

Cette fonctionnalité élevée, le plus souvent supérieure à 5, les rend particulièrement utiles en leur permettant de jouer un rôle de "noeud de réticulation", c'est-à-dire de site de réticulation multiple.

### Composés pourtant au moins deux fonctions à hydrogène(s) labile(s)

Les composés portant au moins deux fonctions à hydrogène labile utilisés dans la présente invention sont également connus. Il peut s'agir de composés organiques de faible masse moléculaire ou bien d'oligomères ou de polymères synthétiques, obtenus par polyaddition, polycondensation et/ou greffage, ou de polymères naturels modifiés chimiquement.

Selon la présente invention, les fonctions à hydrogène labile sont choisies de préférence parmi les fonctions amine primaire (-NH₂), amine secondaire (>NH), hydroxyle (-OH), acide carboxylique (-COOH) ou thiol (-SH).
- Lorsque la fonction à hydrogène labile est une fonction hydroxyle, on peut citer comme familles de composés, les diols et polyols, choisis de préférence parmi :
   1) les diols aliphatiques contenant un groupement hydroxyle tels que 1,2-propanediol, 1,3-propanediol, 1,2-butanediol, 1,3-butanediol, 1,2-pentanediol, 1,4-pentanediol ...
   2) les polyols tels les polyalkylène éthers polyols dont la structure serait décrite par la formule :

      H-(O(CH₂-CHR)ₙ)ₘOH
avec :
- R étant un atome d'hydrogène ou un groupement alkyle contenant de 1 à 5 carbone,
- n étant un nombre entier de 1 à 6, et
- m etant un nombre entier 1 à 100.

On peut en particulier citer les poly(oxytétraetéhylène) glycol, poly (oxy-1,2 propylène) glycol, poly (oxy-1,2 butylène) glycol...
- Lorsque la fonction à hydrogène labile est une fonction amine (NH₂), il peut s'agir d'une diamine, une polyamine, un aminoalcool, un oligomère, ou un polymère à groupes amines, choisis notamment parmi :
   1) les diamines aliphatiques, cycloaliphatiques ou aromatiques ayant notamment de 2 à 60 atomes de carbone, tel que l'éthylènediamine, le 1,2 diaminopropane, le 1,3 diaminopropane, le 1,4 diaminobutane, le 1,2 diamino-2-méthylpropane, le 1,6 diaminohexane, le 1,10 diaminodécane, l'isophoronediamine, l'adamantane diamine, la 2,6 diaminopyridine, ou les diamines obtenues par modification des extrémités d'acides gras dimères,
   2) les amines multifonctionnelles ayant plus de deux groupes amines telles que la mélanine, la 2,4,6 triaminopyrimidine, la 3,3' diaminobenzidine ou la 2,4,5,6 tétraaminopyrimidine,
   3) les oligomères porteurs d'au moins deux groupes amines tels que les diamines de polyalkylèneoxydes JEFFAMINE^{®} de TEXACO (polyétherdiamines),
   4) les dendrimères ou polymères hyperbranchés dont les extrémités de chaînes sont des amines primaires, en particulier les polyamidoamines tels que ceux vendus sous la dénomination STARBUST^{®} par la société DENDRITECH. Les polymères hyperramifiés sont des polycondensats généralement de type polyéthylèneamine obtenus à partir de monomères multifonctionnels, qui ont une structure arborescente similaire à celle des dendrimères mais beaucoup moins régulière que celle-ci.
- Lorsque le composé est un oligomère ou polymère (homopolymère ou copolymère) porteur de fonctions à hydrogène labiles, celles-ci peuvent être situées aux extrémités et/ou latéralement aux chaînes. On peut citer comme familles de composés des copolymères, blocs ou non, acrylique résultant de la polymérisation de monomère comportant des fonctions éthyléniques insaturées telles que l'acide (meth)acrylique avec un monomère alkyl ester de l'acide (méth)acrylique ( méthacrylate de méthyl, méthacrylate d'éthyl...) , des monomères vyniliques tels que le styrene, l'alpha méthyl styrène, le vynil toluène..., les polyuréthanes, les polyesters, les polycondensats de toutes nature porteurs de groupes hydoxyles et en particulier les polyamides obtenus par condensation d'un excès de diamine ou de diacide ou de diol.

Des exemples particuliers de composés porteur de fonctions à hydrogènes labiles sont : les alkylèneglycols en C₁₋₄, le glycérol, le triméthylolpropane, le pentaérythritol, les poly(alkylène en C₁₋₄)glycols tels que le polyéthylèneglycol ou polyproylèneglycol ou des copolymères de ceux-ci, le produit de condensation de propylèneglycol et de triméthylolpropane, l'huile de ricin, le phytantriol, les sucres et carbohydrates tels que le saccharose ou la cellulose, l'éthylènediamine, le 1,3-diaminopropane, la lysine, l'amino-2-méthyl-2-propanol-1, les poly(alkylèneoxy)diamines telles que les produits JEFFAMINE^{®} commercialisés par la société TEXACO, la nitrocellulose, les esters de cellulose, notamment ceux ayant un degré de substitution inférieur à 3, tels que l'acétobutyrate de cellulose et l'acétopropionate de cellulose, les éthers de cellulose tels que l'hydroxyéthylcellulose, la carboxyméthylcellulose, l'hydroxypropylcellulose ou l'éthylcellulose, les résines polyesters, silicones, perfluoropolyéthers, alkydes et polycétones à extrémités hydroxylées, le poly(alcool vinylique) et les copolymères à base d'alcool vinylique, les copolymères d'alcool allylique, les copolymères à base de (méth)acrylate d'hydroxyalkyle en C₂₋₁₀, comme le (méth)acrylate de 2-hydroxyéthyle ou de 2-hydroxypropyle, vendus notamment sous la dénomination JONCRYL^{®} SCX 910 par la société JOHNSON POLYMER ou sous la dénomination CRODOPLAST^{®} AC 5725 par la société CRODA, les copolymères à base de vinylamine ou d'allylamine, les silicones et les perfluoroéthers à extrémités amine primaire ou secondaire, les dendrimères ou polymères hyperramifiés à extrémités hydroxyle ou amine primaire tels que les polyesters hyperramifiés à extrémités hydroxyle commercialisés par la société PERSTORP sous les dénominations BOLTORN^{®} H40 TMP CORE et HBP POLYOL^{®} 3G (décrits dans les demandes internationales WO 93/17060 et WO 96/12754), ou encore les dendrimères de type polyamido-amines à extrémités amine primaire décrits dans l'article de Tomalia, Angewandte Chemie, Int. Engl. Ed. , vol. 29, n° 2, pages 138 -175.

Conviennent également à l'invention, des composés portant au moins deux fonctions de natures distinctes à l'image, par exemple des résines. Il peut notamment s'agir :
- des résines alkydes, et en particulier des oligomères issues de la condensation de composés qui portent des fonctions hydroxyles (alcool, diol, ...) avec des composés qui portent des fonctions acides carboxyliques.
   Ainsi les résines alkydes comportent des fonctions à hydrogènes labiles tels des fonctions hydroxyles et/ou des fonctions amides et/ou des fonctions acides carboxyliques et/ou des fonctions réactives telles que des fonctions éthyléniques insaturées (issus d'un alcool ou un acide gras ayant une chaîne alkyle comportant une fonction ethylènique insaturée). Ces résines peuvent être utilisées comme composé A ou B pour être réticulé en présence d'un composé comportant une fonction réactive telle qu'un isocyanate, une fonction époxyde ou co-réticulée sous UV avec un autre composé comportant des fonctions éthyléniques insaturées.
- Des résines aminoplast qui résultent de la condensation d'un composé ayant une fonction aldéhyde avec un composé ayant une fonction amine ou amide.
   On peut citer par exemple les réactions de condensation du formadéhyde, acétaldéhyde, benzaldéhyde avec une urée, une mélamine. Les résines aminoplast préférées sont issues de la condensation d'une fonction alcool et formadéhyde avec une fonction urée, mélamine.
- Selon une seconde variante de l'invention la réticulation est réalisée par voie photochimique et met en oeuvre au moins deux composés (A) et (B) possédant des fonctions de type doubles liaisons insaturées, en présence d'un photoamorceur.

Selon cette variante, les composés (A) et (B) sont choisis de manière à former un système réactif dont la valence moyenne du système est supérieure à 2.

On appelle valence d'un composé le nombre de liaisons covalentes qu'il peut établir avec ces les composés qui lui sont associés. On défini la valence moyenne comme étant égale au rapport de la somme des valences de tous les composés (A) et (B) divisé par le nombre total de composés (A) et (B) ${V}_{m} = \frac{{\sum }_{ } nivi}{{\sum }_{ } ni}$

Selon cette variante de l'invention, les composés (A) ou (B) peuvent être un composé comportant une fonction de type double liaison insaturée et notamment tels que définis précédemment, et/ou un monomère à insaturation éthylénique.

Comme monomères à insaturation éthylénique ayant au moins un groupement acide ou monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise en particulier l'acide (méth)acrylique et l'acide crotonique, et plus particulièrement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₂₀, plus particulièrement en C₁-C₈, des (méth)acrylates d'aryle, en particulier d'aryle en C₆-C₁₀, des (méth)acrylates d'hydroxyalkyle, en particulier d'hydroxyalkyle en C₂-C₆.

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique sont en particulier des (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acide, on peut par exemple citer les (méth)acrylamides, et notamment les N-alkyl (méth)acrylamides, en particulier d'alkyle en C₂-C₁₂. Parmi les N-alkyl (méth)acrylamides, on peut citer le N-éthyl acrylamide, le N-t-butyl acrylamide et le N-t-octyl acrylamide.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styrèniques, on peut citer le styrène de l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

### Photoamorceur

Les photoamorceurs utilisables dans les compositions cosmétiques de la présente invention sont également connus dans la technique et sont décrits, par exemple dans les articles suivants dont le contenu fait partie intégrante du présent texte : "Les photoinitiateurs dans la réticulation des revêtements", G. Li Bassi, Double Liaison - Chimie des Peintures, n° 361, novembre 1985, pages 34 - 41 ; "Applications industrielles de la polymérisation photoinduite", Henri Strub, L'Actualité Chimique, février 2000, pages 5 - 13 ; et "Photopolymères : considérations théoriques et réaction de prise", Marc, J.M. Abadie, Double Liaison - Chimie des Peintures, n° 435 - 436, 1992, pages 28 - 34.

Ces photoamorceurs englobent :
- les α-hydroxycétones, commercialisées par exemple sous les dénominations IRGACURE^{®} 184, 1173, 2959, 149, 1000, 500 et 4265 par la société CIBA,
- les α-aminocétones, commercialisés par exemple sous les dénominations IRGACURE^{®} 907 et 369 par la société CIBA,
- les chloroacétophénones commercialisées par exemple sous les dénominations TRIGONAL^{®} P par la société AKZO et SANDORAY^{®} 1000 par la société SANDOZ,
- les cétones aromatiques commercialisées par exemple sous les dénominations DAITOCURE^{®} par DAINIPPON, UVECRYL^{®} P 36 par UCB, ESACURE^{®} TZT par LAMBERTI, et QUANTACURE^{®} ITX par WARD BLENKINSOP. On peut également citer les thioxanthones (par exemple ULTRACURE^{®} DXT de SHERWIN WILLIAMS) et les quinones (2-éthylanthraquinone de BASF). Ces cétones aromatiques nécessitent le plus souvent la présence d'un composé donneur d'hydrogène tel que les amines tertiaires et plus particulièrement les alcanolamines,
- les éthers de benzine commercialisés par exemple sous la dénomination ESACURE^{®} EB-3 par la société LAMBERTI et sous la dénomination TRIGONAL^{®} 14 par AKZO,
- les dérivés α-dicarbonyles dont le représentant le plus courant est le benzyldiméthylcétal commercialisé sous la dénomination IRGACURE^{®} 651 par CIBA. D'autres produits commerciaux sont commercialisés par la société LAMBERTI sous la dénomination ESACURE^{®} KBO et par la société WARD BENKINSOP sous la dénomination QUANTACURE^{®} PDO.
- les oxydes d'acylphosphine, tels que par exemple les oxydes de bis-acylphosphine (BAPO) commercialisés par exemple sous la dénomination IRGACURE^{®} 819, 1700, 1800 et 1850 et DAROCUR^{®} 4265 par la société CIBA.

Un groupe particulier de photoamorceurs avantageux selon l'invention est celui des photoamorceurs copolymérisables. Il s'agit de molécules comportant à la fois un groupe photoamorceur capable d'une scission radicalaire photoinduite et au moins une double liaison éthylénique. Les photoamorceurs de ce groupe présentent l'avantage par rapport aux photoamorceurs classiques énumérés ci-dessus de pouvoir être intégrés, par l'intermédiaire de la double liaison, dans le système macromoléculaire. Cette possibilité diminue la concentration de photoamorceurs résiduels libres n'ayant pas subi de coupure radicalaire photoinduite et améliore par conséquent l'innocuité de l'article selon l'invention.

On peut citer à titre d'exemples de tels photoamorceurs copolymérisables les dérivés acrylés de benzophénone commercialisés par la société UCB sous les dénominations EBECRYL^{®} P36 et EBECRYL^{®} P37.

On utilise de préférence dans le système réactif de la présente invention un mélange de photoamorceurs absorbant la lumière à différentes longueurs d'ondes. Il est ainsi possible d'adapter le spectre d'absorption des compositions réticulables au spectre d'émission des sources de lumière utilisées.

La concentration du ou des photoamorceurs utilisée dépend d'un grand nombre de facteurs comme par exemple de la réactivité des différents composants du mélange, de la présence de pigments ou de colorants, de la densité de réticulation recherchée, de l'intensité de la source lumineuse ou du temps d'exposition.

Pour obtenir des propriétés de tenue satisfaisantes, on utilisera généralement une quantité totale de photoamorceur(s) au moins égale à 0,1 % en poids et au plus égale à 10 % en poids, et de préférence comprise entre 0,2 % et 5 % en poids, rapportée au poids total de composés (A) et (B) comportant des doubles liaisons éthyléniques.

Dans une autre variante de l'invention, la réticulation peut être réalisée en mettant en présence des composés (A) et/ou (B) possédant des fonctions (X) et/ou (Y) sous une forme bloquée et susceptible d'être débloquée préalablement ou dans les conditions réactionnelles retenues pour la réticulation.

Ce débloquage peut par exemple être réalisé sous l'action de l'eau en présence ou en absence de catalyseur, notamment lorsque Y est choisi parmi des fonctions amines bloquées et que X est une fonction réactive telle qu'un isocyanate ou une fonction époxyde.

Il peut également être réalisé sous l'action de la chaleur en présence ou non de catalyseur, notamment lorsque X est choisi parmi des fonctions isocyanates bloquées et que Y est une fonction comportant des hydrogènes labiles comme une fonction amine, hydroxyle.

De même, ce déblocage peut être effectué sous l'action d'un rayonnement (par exemple UV, RX, Laser...) éventuellement en présence d'un photoamorceur, lorsque X et Y sont choisis parmi des fonctions réactives tels que des doubles liaisons éthyléniques insaturées.

### Fonctions isocyanates bloquées

Les fonctions isocyanate bloquées susceptibles de réagir, après activation thermique, avec les fonctions à hydrogène labile, peuvent répondre à la formule suivante :

-NH-C(=O)-M

dans laquelle M représente un radical dérivé d'un agent de blocage MH choisi parmi les composés organiques comportant un ou plusieurs, et en particulier un seul atome d'hydrogène labile.

Les agents de blocage doivent être capables d'empêcher la réaction ultérieure des groupes isocyanates à température ambiante, ou plus généralement à une température inférieure à 45 °C, avec toute autre molécule contenant des atomes d'hydrogène labiles, mais doivent permettre cette réaction à une température plus élevée, c'est-à-dire généralement supérieure ou égale à 50 °C, après déblocage thermique de la fonction isocyanate.

Parmi les composés à groupements isocyanate bloqués commercialisés, on peut citer ceux portant les dénominations VESTANAT^{®} B1358A, VESTANAT^{®} B1370, VESTANAT^{®} B1358/100 commercialisés par la société CREANOVA, les dénominations TOLONATE^{®} D2 ou D2R565 commercialisés par la société RHODIA, la dénomination DESMODUR^{®} Z4470 commercialisée par la société BAYER et sous les dénominations TRIXENE^{®} B1 7951 et TRIXENE^{®} B1 7982 commercialisées par la société BAXENDEN.

On peut également utiliser, comme composés (A) ou (B), des composés à fonctions isocyanates "autobloquées", tels que les uréthanne-diones obtenues par dimérisation de 2 molécules de diisocyanates, ou encore les tris((alcoxy en C₁₋₆)-carbonylamino)triazines telles que le produit de condensation de mélanine, de carbonate de diméthyle et de butanol. De tels composés sont notamment vendus sous le nom de CYLINK^{®} 2000 par la société CYTECK.

### Fonctions à hydrogènes labiles bloquées.

Les fonctions à hydrogènes labiles bloquées sont choisies notamment parmi les fonctions amines bloquées sous forme cétimine et aldimine, et les fonctions aminoalcool bloquées sous forme oxazolidine.

Les composés portant les fonctions amines bloquées peuvent notamment être choisis parmi :
a) les polyamines bloquées de formule générale : 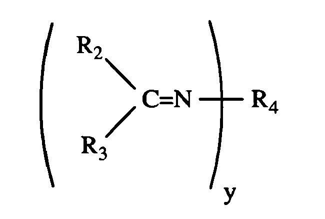 dans laquelle :
   - y varie de 2 à 100,
   - R₂ est égal ou différent de R₃ et choisi parmi un atome d'hydrogène et les groupes alkyle ayant de un à quatre atomes de carbone.

   Des composés à fonctions amines bloquées sous forme cétimine sont notamment vendus sous la dénomination Epikure^{®} H3, Epikure^{®} 3505 par la société SHELL, VESTAMIN^{®} A139 par la société CREANOVA.
   Selon un mode particulier de réalisation de l'invention, le système réactif peut comprendre au moins un composé comportant à la fois des fonctions réactives non bloquées X et des fonctions réactives bloquées Y.
   Des composés à fonctions aminoalcools bloquées sous forme oxazolidine sont notamment décrits dans les documents WO-A-99/07763, JP-A-09-241501, WO-A-96/20231, WO-A-95/14528, US-A-5126421, US-A-4381388, US-A-4504647. Ils sont vendus sous les dénominations INCOZOL^{®} 4, INCOZOL^{®} LV par la société INDUSTRIAL COPOLYMER LTD, HARDENER^{®} OZ par la société BAYER, ZOLDINE^{®} RD-4 par la société ANGUS CHEMICALS CO.
   Les fonctions à hydrogènes labiles bloquées peuvent également être figurées par :
   - des groupements cycliques qui sont susceptibles de réagir par ouverture de cycles comme par exemple :
      ➢ les fonctions anhydrides qui sont susceptibles de générer une fonction carboxylique pour réagir a son tour avec une fonction réactive ou une fonction à hydrogène labiles, les anydrides préférentiellement utilisées sont choisi parmi les anhydrides aliphatique, cycloaliphatique ou aromatiques. Des exemples d'anydride tels que l'anhydride succinique, l'anhydride méthyl succinique, l'anhydride dodecenyl méthyl succinique, l'anhydride phtalique, l'anhydride maléique, l'anhydride itaconique,
      ➢ les fonctions lactone et lactame comme epsilon caprolactone, epsilon caprolactame, et
   - des fonctions acétoacétates : R-O-C=O-CH_{2-C}=O-CH₃ qui sont suceptibles de réagir en présence de formaldéhyde (ou de résines mélamine/formaldéhyde, urée/formaldéhyde, alcoxylés), de fonctions isocyanates, de fonctions époxydes, de doubles liaisons éthyléniques insaturées (réaction de michael) ou de fonctions amines.

   Les systèmes réactifs de la présente invention peuvent contenir un ou plusieurs catalyseurs capables d'accélérer la réaction de réticulation.
   Ces catalyseurs sont choisis notamment parmi les amines tertiaires telles que le diazabicyclo[2,2,2]octane, la quinuclidine et le 3,3,6,9,9-pentaméthyl-2,10-diazabicyclo[4,4,0]déc-1-ène, le chlorure d'étain, les composés organométalliques tels que les acétonylacétates métallliques, les composés organométalliques de l'étain, l'hexanoate de calcium, le 2-éthylhexanoate de calcium, l'octanoate de calcium et le linoléate de calcium, le dibutyldilaurate d'étain, le tris(2-éthylhexanoate) de bismuth et le bis(2-éthylhexanoate) de zinc.
   Selon la présente invention, la concentration des catalyseurs est de préférence comprise entre 0,1 et 5 % en poids, et plus particulièrement entre 0,2 et 3 % en poids, rapportée au poids total du composé porteur des groupes isocyanates bloqués.
   La composition réticulable selon la présente invention peut contenir un ou plusieurs solvants choisis parmi l'eau et les solvants organiques parmi lesquels on peut citer :
b) les cétones liquides à température ambiante telles que la méthyléthylcétone, la méthylisobutylcétone, la diisobutylcétone, l'isophorone, la cyclohexanone et l'acétone,
c) les alcools liquides à température ambiante tels que l'éthanol, l'isopropanol, le diacétone-alcool, le 2-butoxyéthanol ou le cyclohexanol,
d) les glycols liquides à température ambiante tels que l'éthylèneglycol, le propylèneglycol, le pentylèneglycol et le glycérol,
e) les éthers de propylèneglycol liquides à température ambiante tels que le monométhyléther de propylèneglycol, l'acétate de l'éther monométhylique de propylèneglycol, le mono-n-butyléther de dipropylèneglycol,
f) les esters à courte chaîne (comportant au total de 3 à 8 atomes de carbone) tels que l'acétate d'éthyle, l'acétate de méthyle, l'acétate de propyle, l'acétate de n-butyle et l'acétate d'isopentyle,
g) les alcanes liquides à température ambiante tels que le décane, l'heptane, le dodécane, le cyclohexane,
h) les hydrocarbures aromatiques liquides à température ambiante tels que le toluène et le xylène,
i) les silicones liquides à température ambiante et
j) des mélanges de ceux-ci.

La teneur en solvant dans la composition peut aller de 0,1 % à 80 % en poids, par rapport au poids total de la composition initiale avant réticulation, et de préférence de 1 à 60 % en poids.

Selon un mode particulier, la composition réticulable peut être exempte de solvant.

Les compositions réticulables utilisées dans l'invention peuvent en outre comprendre différents additifs sous réserve que ceux-ci ne soient pas susceptibles d'interférer avec la réaction de réticulation.

En particulier, la réticulation peut être effectuée en présence d'au moins un agent filmogène et notamment de la nitrocellulose et/ou des esters de cellulose.

### PARTICULES SOLIDES

Les articles selon l'invention peuvent en outre contenir, et notamment au sein du ou des film(s) réticulé(s), des particules solides notamment en une quantité efficace pour améliorer leur tenue et en particulier leur résistance à l'usure de l'article. Ces particules peuvent être formées à partir de matériaux choisis parmi des matériaux minéraux polymères ou non polymères, des matériaux organiques polymères ou non polymères qui peuvent être métalliques, des matériaux composites et leurs mélanges.

En particulier, les particules pouvant être utilisées dans les articles selon l'invention peuvent comprendre des éléments minéraux ou des composés connus dans le domaine. Des particules appropriées peuvent être formées à partir de matériaux céramiques, de matériaux métalliques ou de leurs mélanges. Des matériaux céramiques appropriés comprennent des oxydes métalliques, des nitrures de métaux, des carbures de métaux, des sulfures de métaux, des silicates de métaux, des nitrures de métaux, des carbonates de métaux et leurs mélanges.

Des matériaux minéraux non polymères peuvent être choisis parmi le graphite, des métaux, des oxydes, des carbures, des nitrures, des porures, des sulfures, des silicates, des carbonates, des sulfates, et des hydroxydes. A titre illustratif, on peut citer comme exemple d'oxyde minéral de l'oxyde de zinc. A titre illustratif, on peut citer comme exemple de sulfure inorganique, le disulfure de molybdène, le disulfure de tentane, le disulfure de tungstène et le disulfure de zinc. Comme exemple non limitatif de silicates minéraux, on peut citer par exemple les silicates d'aluminium et de magnésium tels que la vermiculite. Comme exemples de métaux appropriés, on peut citer le molybdène, le platine, le palladium, le nickel, l'aluminium, l'or, le fer, l'argent et leurs alliages et mélanges.

Selon un mode de réalisation particulier, il peut s'agir de particules choisies parmi de la silice pyrogénée notamment la famille dénommée Aérosil^{®} de Degussa, de la silice amorphe, de la silice colloïdale, de l'alumine, de l'alumine colloïdale, du dioxyde de titane, de l'oxyde d'atrium, du zirconium colloïdale, des argiles et leurs mélanges.

Conviennent tout particulièrement à titre d'argiles, les phyllosilicates intercalés voire exfoliés. Le terme « intercalé », signifie que ces phyllosilicates ont été traités avec des composés organiques ou inorganiques en vue d'introduire des molécules de ces composés dans les espaces interfoliaires. Les phyllosilicates exfoliés désignent les plaquettes séparées obtenues généralement par cisaillement des phyllosilicates intercalés. De tels matériaux sont notamment décrits dans les documents WO 93/04118, US 5 721 306 et US 6 500 411.

Les particules peuvent également être formées à partir de matériau polymère minéral. Comme matériau polymère minéral, on peut citer notamment les polyphosphasènes, polysilanes, polysiloxane, les sulfures polymériques, les silinium polymériques, les silicones et leurs mélanges. Un exemple particulier de particules formées à partir d'un matériau polymère minéral sont les particules commercialisées sous la dénomination de Tospearl par la Société Toshiba Silicone Company qui sont des particules de siloxane réticulées.

Les particules peuvent également être formées de matériaux organiques non polymériques. Comme matériau organique non polymérique, on peut citer notamment des stéarates, tels que le stéarate de zinc et le stéarate d'aluminium, le diamant, le charbon noir et le stéaramide.

Les particules peuvent également être des particules creuses formées à partir de matériaux polymères choisis parmi des matériaux minéraux polymères ou non polymères, des matériaux organiques polymères ou non polymères ou des matériaux composites et leurs mélanges. Comme exemple de matériaux appropriés formant des particules creuses, on peut citer notamment des particules creuses de verre.

Comme autre exemple de particules incluant des silices colloïdales, on peut citer notamment les particules commercialisées par la société Nissan Chemical Company sous la dénomination « Organosilicasol^{®} » tel que l'Organosilicasol MT-ST et par la société Clariant Corporation sous la dénomination « High link^{®} », des alumines colloïdales telles que celles commercialisées par la société Nalco Chemical sous la dénomination « Nalco 8667^{®} » et des zirconia colloïdales telles que celles commercialisées par la société Nissan Chemical Company sous la dénomination « HIT-32M^{®} ».

Plus particulièrement, ces particules peuvent être choisies parmi le talc, le stéarate de zinc, le mica, le kaolin, les poudres de polyamide (Nylon^{®}) (Orgasol^{®} de chez Atochem), les poudres de polyéthylène, les poudres de polymères de tétrafluoroéthylène (Téflon^{®}), l'amidon, le nitrure de bore, des microsphères polymériques telles que celles de chlorure de polyvinylidène/acrylonitrile comme l'Expancel^{®} (Nobel Industrie), de copolymères d'acide acrylique (Polytrap^{®} de la société Dow Corning) et les microbilles de résine de silicone (Tospearls^{®} de Toshiba, par exemple), les organopolysiloxanes élastomères, et leurs mélanges.

En particulier, la teneur en particules, dans les articles selon l'invention est inférieure à 40 % en poids, notamment inférieure à 25 %, plus particulièrement inférieure à 15 % et voire inférieure à 10 % en poids par rapport au poids total de l'article.

### MATERIAU ADHESIF

L'article selon l'invention possède une face externe adhésive. Une telle face adhésive est obtenue de façon générale grâce à la présence d'au moins une couche d'au moins un matériau adhésif.

Par « matériau », on entend au sens de la présente invention un polymère ou un système polymérique pouvant comprendre un ou plusieurs polymères de natures différentes. Ce matériau adhésif peut se présenter sous forme d'une solution de polymère ou d'une dispersion de particules de polymères dans un solvant. Ce matériau adhésif peut en outre contenir un plastifiant. Ce matériau adhésif doit présenter un certain pouvoir collant défini par ses propriétés viscoélastiques.

Les propriétés viscoélastiques d'un matériau sont classiquement définies par deux valeurs caractéristiques qui sont les suivantes :
- le module élastique qui représente le comportement élastique du matériau pour une fréquence donnée et qui est classiquement noté G',
- le module visqueux qui représente le comportement visqueux du matériau pour une fréquence donnée et qui est classiquement noté G".

Ces grandeurs sont notamment définies dans le "Handbook of Pressure Sensitive Adhesive Technology" 3rd édition, D. Satas, chap. 9, p. 155 à 157.

Les matériaux adhésifs utilisables selon la présente invention présentent des propriétés viscoélastiques qui sont mesurées à une température de référence de 35 °C et dans un certain intervalle de fréquences.

Dans le cas de matériaux adhésifs sous forme de solution ou de dispersion de polymère dans un solvant volatil (tel que l'eau, un ester court, un alcool court, de l'acétone ...), on mesure les propriétés viscoélastiques de ce matériau dans des conditions dans lesquelles il présente une teneur en solvant volatil inférieure à 30 %, et en particulier une teneur en solvant volatil inférieure à 20 %.

On mesure en particulier le module élastique du matériau à trois fréquences différentes :
- à faible fréquence, soit à 2.10⁻² Hz,
- à une fréquence intermédiaire, soit à 0,2 Hz,
- à haute fréquence, soit à 2 Hz,
   et le module visqueux à la fréquence de 0,2 Hz.

Ces mesures permettent d'évaluer l'évolution du pouvoir collant du matériau adhésif au cours du temps.

Ces propriétés viscoélastiques sont mesurées lors d'essais dynamiques sous sollicitations sinusoïdales de faible amplitude (petites déformations) réalisés à 35 °C sur une plage de fréquence allant de 2.10⁻² à 20 Hz sur un rhéomètre de type "Haake RS50^{®}" sous une sollicitation de torsion/cisaillement, par exemple en géométrie cône-plan (par exemple avec un angle du cône de 1°).

Avantageusement, ledit matériau adhésif répond aux conditions suivantes :
- G'(2 Hz, 35 °C) ≥ 10³ Pa, et
- G'(35 °C) ≤ 10⁸ Pa, en particulier G'(35 °C) ≤ 10⁷ Pa,
- G' (2.10⁻² Hz, 35°C) ≤ 3.10⁵ Pa,
dans lesquelles :
- G'(2 Hz, 35 °C) est le module élastique de cisaillement dudit matériau adhésif, mesuré à la fréquence de 2 Hz et à la température de 35 °C,
- G'(35 °C) est le module élastique de cisaillement dudit matériau adhésif, mesuré à la température de 35 °C, pour toute fréquence comprise entre 2.10⁻² et 2 Hz,
- G'(2.10⁻² Hz, 35 °C) est le module élastique de cisaillement dudit matériau adhésif, mesuré à la fréquence de 2.10⁻² Hz et à la température de 35 °C.

Dans une forme particulière de l'invention, le matériau adhésif répond également à la condition suivante :
- G"/G' (0,2 Hz, 35 °C) ≥ 0,35.
dans laquelle :
- G"(0,2 Hz, 35 °C) est le module visqueux de cisaillement dudit matériau adhésif, mesuré à la fréquence de 0,2 Hz et à la température de 35 °C,
- G'(0,2 Hz, 35 °C) est le module élastique de cisaillement dudit matériau adhésif, mesuré à la fréquence de 0,2 Hz et à la température de 35°C.

Dans une forme particulière de l'invention, on a :
- G'(2 Hz, 35 °C) ≥ 5.10³ Pa, et en particulier, G'(2 Hz, 35 °C) ≥ 10⁴ Pa.

Dans une autre forme particulière de l'invention, on a :
- G'(2.10⁻² Hz, 35°C) ≤ 5.10⁴ Pa.

En particulier, les matériaux adhésifs selon l'invention répondent aux quatre conditions suivantes :
- G'(2 Hz, 35 °C) ≥ 10⁴ Pa, et
- G'(35 DC) ≤ 10⁸ Pa, en particulier G'(35 °C) ≤ 10⁷ Pa,
- G'(2.10⁻² Hz, 35 °C) ≤ 5.10⁴ Pa, et
- G"/G'(0,2 Hz, 35 °C) ≥ 0,35.

D'une manière générale, l'adhésif est tel que ledit article ne peut être ôté par pelage lorsqu'il est appliqué à la surface d'un ongle synthétique ou naturel après au moins 24 heures de pose.

Plus particulièrement, les matériaux adhésifs selon l'invention peuvent être choisis parmi les adhésifs de type "Pressure Sensitive Adhesives" (adhésifs sensibles à la pression) par exemple, comme ceux cités dans le "Handbook of Pressure Sensitive Adhesive Technology" 3^{rd} édition, D. Satas.

Les matériaux adhésifs selon l'invention sont notamment des polymères choisis parmi les copolymères blocs ou statistiques comprenant au moins un monomère ou une association de monomères dont le polymère résultant à une température de transition vitreuse inférieure à la température ambiante (25 °C), ces monomères ou associations de monomères pouvant être choisis parmi le butadiène, l'éthylène, le propylène, l'isoprène, l'isobutylène, une silicone, et leurs mélanges. Des exemples de tels matériaux sont les polymères blocs de type styrène-butadiène-styrène, styrène-(éthylène-butylène)-styrène, styrène-isoprène-styrène comme ceux vendus sous les dénominations commerciales "Kraton^{®}" de SHELL CHEMICAL Co. ou de "Vector^{®}" d'EXXON.

Les matériaux adhésifs selon l'invention sont en particulier des polymères adhésifs choisis parmi :
- les polyuréthannes,
- les polymères acryliques,
- les silicones,
- les gommes butyliques, notamment parmi les polyisobutylènes,
- les polymères éthylène-acétate de vinyle,
- les polyamides éventuellement modifiés par des chaînes grasses,
- les gommes naturelles,
- et leur mélanges.

Il peut en particulier s'agir de copolymères adhésifs dérivant de la copolymérisation de monomères vinyliques avec des entités polymériques comme par exemple ceux décrits dans le brevet US 6 136 296. Sont également susceptibles de convenir à l'invention, les copolymères adhésifs décrits dans le brevet US 5 929 173 possédant un squelette polymérique, de Tg variant de 0°C à 45°C, greffés par des chaînes dérivant de monomères acryliques et/ou méthacryliques et possédant en revanche une Tg variant de 50 °C à 200 °C.

Les matériaux adhésifs sont par exemple choisis parmi les polyisobutylènes présentant une masse molaire relative Mv supérieure ou égale à 10 000 et inférieure ou égale à 150 000. En particulier, cette masse molaire relative est supérieure ou égale à 18 000 et inférieure ou égale à 150 000.

Comme produits commerciaux convenant particulièrement bien à la présente invention, on peut citer les polyisobutylènes de masses molaires relatives Mv respectives 40 000, 55 000 et 85 000 vendus sous les dénominations commerciales respectives "Oppanol B 10^{®}", "Oppanol B 12^{®}" et "Oppanol B 15^{®}" par la société BASF, et leurs mélanges.

Le matériau adhésif dans l'article conforme à l'invention est généralement sous la forme d'une couche ayant une épaisseur de 1 micron à 100 microns et en particulier de 1 micron à 50 microns, de préférence de 1 micron à 25 microns.

Selon un mode de réalisation particulier de l'invention, la couche formée par le matériau adhésif est directement en contact avec le film réticulé.

Avantageusement le matériau adhésif et le film présentent une compatibilité grâce à leur nature chimique. En effet, comme précisé précédemment, le solvant de l'adhésif est susceptible de conduire à une augmentation de masse du film réticulé mis à son contact, notamment d'au moins 10 % en poids par rapport au poids initial du film réticulé. Cette augmentation se traduit plus précisément par une reprise en masse du film.

Toutefois, comme précisé précédemment, l'article peut également posséder un film de vernis entre la couche adhésive et le film réticulé. Cette couche intermédiaire entre la couche de matériau adhésif et le film peut être obtenue par évaporation de la phase aqueuse de la dispersion aqueuse ou organique d'un dispersion ou solution d'au moins un polymère filmogène. Une telle couche peut être en particulier constituée d'un film de vernis notamment coloré. Une telle architecture est particulièrement avantageuse en terme de durée dans le temps. Le film réticulée protège efficacement le film de vernis vis-à-vis des chocs et donc en prolonge significativement la tenue dans le temps.

### AUTRES ADDITIFS

L'article peut en outre comprendre notamment au sein de son film réticulé, au moins une matière colorante, notamment tel qu'un pigment et/ou au moins une nacre et/ou au moins des paillettes classiquement utilisés dans les compositions cosmétiques. Bien entendu, certains de ces composés sont susceptibles d'être également illustrés par les particules solides discutées précédemment.

Par pigments, il faut comprendre des particules blanches ou colorées, minérales ou organiques, insolubles dans une solution aqueuse, destinées à colorer et/ou opacifier le film résultant. Par nacres, il faut comprendre des particules irisées, notamment produites par certains mollusques dans leur coquille ou bien synthétisées. Ces nacres servent notamment à modifier la texture du film attendu.

Les pigments peuvent être présents à raison de 0,01 à 15 % en poids, notamment de 0,01 à 10 % en poids, et en particulier de 0,02 à 5 % en poids, par rapport au poids total de l'article. Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome.

Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Les nacres peuvent être présentes à raison de 0,01 à 15 % en poids, de préférence de 0,01 à 10 % en poids, et mieux de 0,02 à 5 % en poids par rapport au poids total de l'article. Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Ces nacres, de même que les paillettes ou autres composés à effets réfléchissants, sont particulièrement intéressantes pour exacerber la brillance naturelle du film réticulé.

L'article selon l'invention peut comprendre également des colorants hydrosolubles ou liposolubles en une teneur allant de 0,01 à 10 % en poids, notamment allant de 0,01 à 5 % en poids par rapport au poids total de du film Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine Les colorants hydrosolubles sont par exemple le jus de betterave, le bleu de méthylène.

Il peut également contenir des ingrédients couramment utilisés en cosmétique et plus spécialement dans le domaine cosmétique et/ou soin des ongles. Ils peuvent notamment être choisis parmi les vitamines, les oligo-éléments, les adoucissants, les séquestrants, les agents alcalinisants ou acidifiants, les polymères filmogènes, les agents d'étalement, les agents mouillants, les agents épaississants, les agents dispersants, les anti-mousses, les conservateurs, les filtres UV, les actifs, les agents hydratants, les parfums, les neutralisants, les stabilisants, les antioxydants et leurs mélanges.

Ainsi, lorsque les articles selon l'invention sont plus particulièrement destinés au soin des ongles naturels, elles peuvent notamment incorporer, à titre d'actifs, des agents durcissants pour matières kératiniques, des actifs agissant sur la croissance de l'ongle comme par exemple le méthyl sulfonyle méthane, et/ou des actifs pour traiter des affections diverses localisées au niveau de l'ongle, comme par exemple l'onichomycose.

Les quantités de ces différents ingrédients sont celles classiquement utilisées dans ce domaine et par exemple de 0,01 à 20 % et notamment de 0,01 à 10 % en poids, par rapport au poids total de l'article.

L'article conforme à l'invention présente généralement une épaisseur de 1 micron à 500 microns, notamment de 1 micron à 300 microns et en particulier de 1 micron à 200 microns.

Comme mentionné précédemment, l'article conforme à l'invention est revêtu au moins sur sa face externe adhésive par un support amovible.

Un tel support peut être de toute nature compatible avec le fait que bien qu'il soit en contact avec un matériau adhésif, il peut néanmoins en être séparé.

Le support amovible défini précédemment peut notamment se présenter sous la forme d'une couche protectrice consistant par exemple en un film, en particulier en un film plastique ou un papier ou une structure textile de type feuille.

Avantageusement, ce support est constitué d'un matériau transparent afin de prévenir toute erreur dans le choix de la couleur. Il peut être constitué d'une ou plusieurs couches pouvant être de nature différente. Par exemple, il peut s'agir d'une feuille de papier revêtue avec l'un des plastiques mentionnés ci-après.

Comme film plastique approprié pouvant être par exemple utilisé dans l'article conforme à l'invention, on peut citer des films faits de polyesters, par exemple des téréphtalates de polyéthylène, des téréphtalates de polybutylène ou sébaçates de polyéthylène ou faits de polyéthylène, polypropylène ou polyamides tels que de l'adipate de polyhexaméthylène, du polycaprolactame ou poly(acide oméga-ω-undécanoïque amide). En raison de leurs caractéristiques de surface, ces plastiques ne sont bien entendu pas amovibles en tant que tels. Afin de leur conférer cette caractéristique, il est nécessaire de procéder à un traitement de surface à l'aide de substances appropriées telles qu'un traitement par silicones ou, de façon particulièrement avantageuse, par un traitement avec des sels d'acides gras à longues chaînes tels que par exemple en C₁₂ à C₂₂, ces acides étant saturés ou pouvant contenir jusqu'à trois liaisons oléfines, et au moins des métaux divalents, en particulier des sels de métaux lourds de transition de ce type et en particulier des sels de chrome.

La structure textile de type de feuille peut être un tissé ou un non tissé.

Selon un mode de réalisation particulier, l'article conforme à l'invention est revêtu sur ses deux faces avec un support amovible, identique ou différent.

L'article conforme à la présente invention peut se présenter sous diverses formes telles qu'une étoile, un carré, un rond, etc....

La présente invention concerne également comme indiqué précédemment un procédé de préparation d'un article souple de maquillage et/ou de soin des ongles. Un tel article peut notamment être obtenu avec le dispositif décrit dans le brevet US 4 903 840.

Plus précisément, ce procédé comprend une étape de réticulation d'au moins deux composés (A) et (B) tels que définis ci-dessus de façon à obtenir un film. Cette réticulation peut être réalisée selon des méthodes conventionnelles bien connues de l'homme du métier.

Le matériau adhésif est généralement déposé sous la forme d'une couche de matériau présentant une épaisseur pouvant aller de 0,5 microns à 200 microns, et en particulier de 1 micron à 100 microns.

L'article obtenu, et en particulier l'excès de film, est ensuite généralement découpé, avant ou après son application, selon la taille et la forme désirée avec de petits ciseaux, un coupe ongles ou en grattant le film.

La présente invention concerne également un procédé de maquillage des ongles dans lequel on applique l'article tel que défini précédemment.

Le maquillage ainsi obtenu peut être éliminé à l'aide des démaquillants usuels dans le domaine des vernis à ongles.

## Revendications

1. Article souple destiné à être appliqué sur les ongles et/ou faux ongles pour leur maquillage et/ou leur soin comportant :
- au moins une couche adhésive permettant la fixation de l'article sur l'ongle et
- au moins un film réticulé,
**caractérisé en ce qu'**il possède une teneur en matière sèche supérieure à 80 % par rapport à son poids total et **en ce qu'**il peut être démaquillé à l'aide d'un dissolvant acétate d'alkyle.

2. Article souple destiné à être appliqué sur les ongles et/ou faux ongles pour leur maquillage et/ou leur soin comportant :
- au moins une couche adhésive permettant la fixation de l'article sur l'ongle et
- au moins un film réticulé;
**caractérisé en ce qu'**il possède une teneur en matière sèche supérieure à 80 % par rapport à son poids total et **en ce qu'**il possède une déformation à la rupture εᵣ supérieur ou égale à 5 %.

3. Article selon la revendication 1 ou 2 **caractérisé en ce qu'**il comprend en outre entre la couche adhésive et le film réticulé au moins un film de vernis coloré.

4. Article selon l'une quelconque des revendications 1 à 3, **caractérisé en ce que** ledit film réticulé est transparent.

5. Article selon la revendication 3 ou 4 **caractérisé en ce que** le film de vernis est obtenu par réticulation ou évaporation de la phase solvant organique ou aqueuse d'une solution ou dispersion d'au moins un polymère filmogène.

6. Article selon la revendication 5, **caractérisé en ce que** le polymère filmogène est choisi parmi la nitrocellulose ou des esters de cellulose.

7. Article selon l'une quelconque des revendications précédentes, **caractérisé en ce qu'**il possède une teneur en matière sèche supérieure à 85 % en poids par rapport à son poids total.

8. Article selon l'une quelconque des revendications précédentes, **caractérisé en ce qu'**il possède une reprise à l'eau portée à 25 °C inférieure ou égale à 20 %, notamment inférieure ou égale à 16 %, et en particulier inférieure ou égale à 10 %.

9. Article selon l'une quelconque des revendications précédentes, **caractérisé en ce qu'**il possède un module de conservation E' supérieur ou égal à 1 MPa, notamment allant de 1 MPa à 5000 MPa, en particulier supérieur ou égal à 5 MPa, notamment allant de 5 à 1000 MPa, et plus particulièrement supérieur ou égal à 10 MPa par exemple allant de 10 à 500 MPa à une température de 30 °C et une fréquence de 0,1 Hz.

10. Article selon l'une quelconque des revendications précédentes, **caractérisé en ce qu'**il possède une déformation à la rupture εᵣ allant de 5 à 500 % et/ou une énergie à rupture par unité de volume Wᵣ supérieure ou égale à 0,2 J/cm³, notamment allant de 0,2 à 100 J/cm³.

11. Article selon l'une quelconque des revendications précédentes, **caractérisé en ce que** la réticulation est réalisée par voie thermique, photochimique et/ou chimique, en présence ou non d'un catalyseur.

12. Article selon l'une quelconque des revendications précédentes, **caractérisé en ce que** ladite réticulation est de type polyaddition et/ou polycondensation.

13. Article selon la revendication 11 ou 12, **caractérisé en ce que** le film réticulé dérive de la réticulation d'un système réactif formé par:
- au moins un premier composé (A) comprenant au moins deux fonctions X,
- au moins un deuxième composé (B) comprenant au moins deux fonctions Y réactives vis-à-vis des fonctions X.

14. Article selon la revendication 13, **caractérisé en ce que** ledit système réactif possède une fonctionnalité moyenne (nombre total des fonctions X et Y/nombre total des molécules de composés (A) et (B)) supérieure à 2 en particulier supérieure ou égale à 2,2 et notamment variant de 2,5 à 100.

15. Article selon la revendication 13 ou 14, **caractérisé en ce que** ledit composé (A) et/ou composé (B) est organique et est notamment un oligomère, polymère ou un copolymère.

16. Article selon la revendication 13, 14 ou 15, **caractérisé en ce que** ledit composé (A) et/ou composé (B) est inorganique et possède en surface lesdites fonctions X. ou Y.

17. Article selon l'une quelconque des revendications 13 à 16, **caractérisé en ce que** lesdites fonctions X et Y sont choisies parmi des fonctions dites réactives et des fonctions comportant au moins un hydrogène labile.

18. Article selon l'une quelconque des revendications 13 à 17, **caractérisé en ce que** les fonctions réactives sont choisies parmi les fonctions isocyanates, époxydes et les doubles liaisons éthyléniques.

19. Article selon la revendication 17 ou 18, **caractérisé en ce que** les fonctions à hydrogène(s) labile(s) sont du type carboxylique, alcool notamment phénolique, amine primaire ou secondaire, amide, aminoalcool et/ou thiol.

20. Article selon l'une quelconque des revendication 12 à 13, **caractérisé en ce que** ladite réticulation met en oeuvre au moins un premier type de composé comportant des fonctions réactives et un second type de composé comportant des fonctions à hydrogène(s) labile(s).

21. Article selon la revendication 20, **caractérisé en ce que** les fonctions réactives sont des fonctions époxydes et/ou isocyanates.

22. Article selon l'une quelconque des revendications 18 à 21, **caractérisé en ce que** les composés porteurs de fonctions réactives de type isocyanate sont choisis parmi les diisocyanates, triisocyanates et polyisocyanates aliphatiques, cycloaliphatiques ou aromatiques de masse moléculaire inférieure à 10 000.

23. Article selon la revendication 22, **caractérisé en ce que** lesdits composés sont choisis parmi :
- le 1,4-tétraméthylène diisocyanate, le 1,6-hexaméthylènediisocyanate, le 2,6- et le 2,4-toluènediisocyanate, le diphénylméthanediisocyanate, et l'isophoronediisocyanate,
- les triisocyanates de formule : 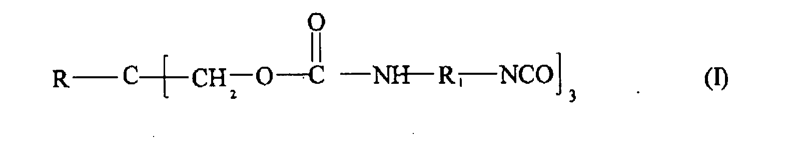 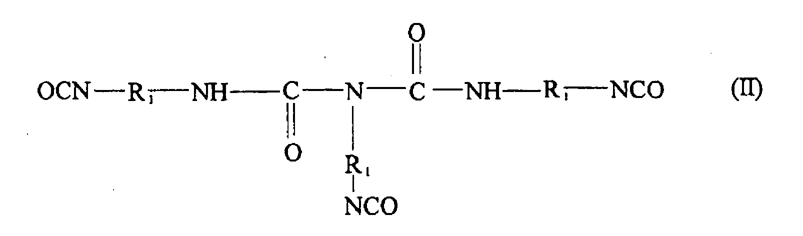 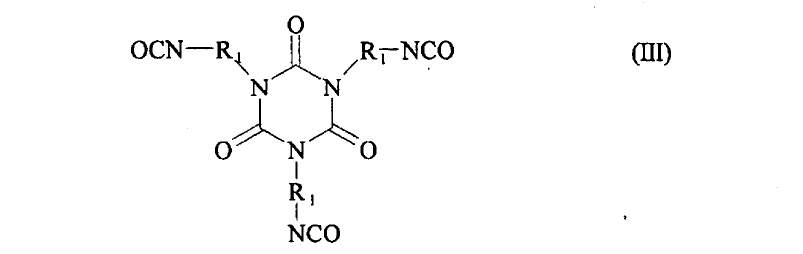 avec R étant un radical alkyle comprenant de 1 à 30 atomes de carbone, et chaque R₁ représentant indépendamment un radical divalent hydrocarboné linéaire, ramifié ou cyclique ayant de 2 à 30 atomes de carbone.
- les polycondensats à groupes isocyanate terminaux ou latéraux tels que les polyuréthannes, polyurées, les polyesters, polyamides, polyépoxy, polyéthers et/ou perfluoropolyéthers, et
- les polymères résultant de la copolymérisation de monomères vinyliques, allyliques et/ou (méth)acryliques et de comonomères à insaturation éthylénique comportant une fonction isocyanate libre.

24. Article selon l'une quelconque des revendications 18 à 23; **caractérisé en ce que** les composés porteurs de fonctions réactives de type époxyde sont choisis parmi :
- le diglycidyl éther de bisphénol A,
- les résines diépoxy,
- les résines époxyester à extrémités α, ω-diépoxy,
- les résines époxyéthers à extrémités α, ω-diépoxy,
- les huiles naturelles ou synthétiques portant au moins deux groupes époxydes,
- les oligomères ou polymères résultant de la copolymérisation de monomères insaturés ou vinyliques, allyliques et/ou (méth)acryliques, et de comonomères à insaturation éthylénique comportant une fonction époxyde libre, et
- les polycondensats à groupes époxy terminaux et/ou latéraux.

25. Article selon l'une quelconque des revendications 18 à 24, **caractérisé en ce que** les composés porteurs de fonction à hydrogène(s) labile(s) sont choisis parmi :
- les diols aliphatiques,
- les polyols,
- les diamines aliphatiques, cycloaliphatiques ou aromatiques,
- les amines multifonctionnelles ayant plus de deux groupes amines,
- les oligomères porteurs d'au moins deux groupes amines,
- les résines alkydes, et
- les dendrimères ou polymères hyperbranchés dont les extrémités de chaînes sont des amines primaires.

26. Article selon l'une quelconque des revendications 1 à 11, **caractérisé en ce que** ladite réticulation est réalisée par voie photochimique et met en oeuvre au moins deux composés (A) et (B) possédant des fonctions de type doubles liaisons insaturées en présence d'un photoamorceur.

27. Article selon la revendication 26, **caractérisé en ce que** (A) et (B) sont choisis de manière à former un système réactif dont la valence moyenne est supérieure à 2.

28. Article selon la revendication 27, **caractérisé en ce que** les composés (A) et (B) sont choisis parmi :
a) Les polyesters à insaturation(s) éthylénique(s),
b) Les polyesters à groupes (meth)acrylate latéraux et/ou terminaux,
c) Les polyuréthannes et/ou polyurées, à groupes (méth)acrylate
d) Les poly(méth)acrylates d'(alkyle en C₁₋₅₀) comportant au moins deux fonctions à double liaison éthylénique portées par les chaînes hydrocarbonées latérales et/ou terminales,
e) Les polyorganosiloxanes à groupes (méth)acrylate ou (méth)acrylamide,
f) Les dendrimères et polymères hyperramifiés portant des groupes terminaux (méth)acrylate ou (méth)acrylamide, et
g) Les monomères à insaturation éthylénique.

29. Article selon l'une quelconque des revendications précédentes, **caractérisé en ce que** ledit film comprend en outre un catalyseur.

30. Article selon l'une quelconque des revendications 1 à 29, **caractérisé en ce qu'**il comprend en outre au moins une matière colorante, notamment des pigments, des nacres et ou des paillettes.

31. Article selon l'une quelconque des revendications précédentes, **caractérisé en ce qu'**il comprend en outre des particules solides organiques ou inorganiques.

32. Article selon l'une quelconque des revendications précédentes, **caractérisé en ce que** ladite couche adhésive comporte au moins un matériau adhésif.

33. Article selon l'une quelconque des revendications précédentes, **caractérisé en ce que** ledit matériau adhésif est tel que ledit article ne peut être ôté par pelage lorsqu'il est appliqué à la surface d'un ongle synthétique ou naturel après au moins 24 heures de pose.

34. Article selon la revendication 32 ou 38, **caractérisé en ce que** ledit matériau adhésif est choisi parmi les copolymères dérivant de la copolymérisation de monomères vinyliques avec des entités polymériques, des copolymères possédant un squelette polymérique, de Tg variant de 0 °C à 45 °C, greffés par des chaînes dérivant de monomères acryliques et/ou méthacryliques et possédant en revanche une Tg variant de 50 °C à 200 °C et les polyisobutylènes présentant une masse molaire relative Mv supérieure ou égale à 10 000 et inférieur ou égale à 150 000.

35. Article selon l'une quelconque des revendications précédentes, **caractérisé en ce que** ladite couche adhésive est revêtue en surface d'un support amovible.

36. Article selon la revendication précédente, **caractérisé en ce que** ledit support amovible est constitué d'un film plastique modifié par un traitement de surface par silicone ou par des sels d'acides gras en C₁₂ à C₂₂.

37. Produit de maquillage et/ou de soins des ongles et/ou des faux ongles comprenant dans un conditionnement sensiblement étanche à l'air, au moins un article souple destiné à être appliqué sur les ongles et/ou faux ongles pour leur maquillage et/ou leur soin comportant :
- au moins une couche adhésive permettant la fixation de l'article sur l'ongle et
- au moins un film réticulé, pouvant être démaquillé à l'aide d'un dissolvant, ledit film réticulé pouvant être conforme à l'une quelconque des revendications 4 à 6 et 11 à 29 et ledit article pouvant en outre comprendre les caractéristiques selon les revendications 30 à 36, le conditionnement étant tel que ledit article s'y trouve conservé sous une forme partiellement sèche.

38. Produit selon la revendication 37, **caractérisé en ce que** ledit article possède une teneur en matière sèche inférieure à 80 %, en particulier inférieure à 75 % et plus particulièrement inférieure à 70 % en poids par rapport au poids total dudit article.

39. Produit selon la revendication 37 ou 38, **caractérisé en ce que** le conditionnement comporte un réservoir apte à contenir de manière étanche ledit article.

40. Procédé de préparation d'un article souple de maquillage et/ou de soin des ongles comprenant au moins les étapes consistant à superposer sur un support amovible :
a) au moins une couche d'une composition à base d'au moins un matériau adhésif, et
b) au moins une couche d'une composition réticulable, la réticulation de ladite composition étant réalisée consécutivement au dépôt de celle-ci de manière à obtenir un film réticulé.

41. Procédé selon la revendication 40, **caractérisé en ce que** l'on forme en outre, entre la couche adhésive et le film réticulé, un film de vernis coloré.

42. Procédé selon la revendication 41, **caractérisé en ce que** le film de vernis est obtenu par réticulation et/ou évaporation de la phase solvant organique ou aqueuse d'une solution ou dispersion d'au moins un polymère filmogène.

43. Procédé selon l'une quelconque des revendications 40 à 42, **caractérisé en ce que** la réticulation est de type polyaddition et/ou polycondensation comme définie en revendication 12 à 25.

44. Procédé selon l'une quelconque des revendications 40 à 42, **caractérisé en ce que** la réticulation est réalisée par voie photochimique comme défini en revendication 25 à 27.

45. Procédé selon l'une quelconque des revendications 40 à 44, **caractérisé en ce que** le matériau adhésif est tel que défini en revendication 33 ou 34.

46. Procédé de préparation d'un produit selon l'une quelconque des revendications 37 à 38, comprenant les étapes consistant à superposer sur un support amovible :
a) au moins une couche d'une composition à base d'au moins un matériau adhésif,
b) au moins une couche d'une composition réticulable, la réticulation dudit film étant effectuée consécutivement au dépôt de ladite composition,
c) si nécessaire le séchage partiel de l'article ainsi obtenu, et
d) le conditionnement dudit article à un état partiellement sec au sein d'un conditionnement sensiblement étanche à l'air.

47. Procédé de maquillage des ongles utilisant un article tel que défini en revendication 1 à 36 comprenant le fait d'appliquer sur un ongle naturel ou synthétique, la face adhésive dudit article.

## Claims

1. Flexible article intended to be applied to the nails and/or false nails for making them up and/or caring for them, comprising:
- at least one adhesive layer for fixing the article to the nail, and
- at least one crosslinked film,
**characterized in that** it has a solids content of greater than 80% relative to its total weight and **in that** it can be removed using an alkyl acetate solvent.

2. Flexible article intended to be applied to the nails and/or false nails for making them up and/or caring for them, comprising:
- at least one adhesive layer for fixing the article to the nail, and
- at least one crosslinked film,
**characterized in that** it has a solids content of greater than 80% relative to its total weight and **in that** it has an ultimate strain εᵣ of greater than or equal to 5%.

3. Article according to Claim 1 or 2,
**characterized in that** it also comprises, between the adhesive layer and the crosslinked film, at least one film of colored varnish.

4. Article according to any one of Claims 1 to 3, **characterized in that** said crosslinked film is transparent.

5. Article according to Claim 3 or 4,
**characterized in that** the film of varnish is obtained by crosslinking or evaporation of the organic or aqueous solvent phase of a solution or dispersion of at least one film-forming polymer.

6. Article according to Claim 5, **characterized in that** the film-forming polymer is chosen from nitrocellulose and cellulose esters.

7. Article according to any one of the preceding claims, **characterized in that** it has a solids content of greater than 85% by weight relative to its total weight.

8. Article according to any one of the preceding claims, **characterized in that** it has an uptake of water brought to 25°C of less than or equal to 20%, especially less than or equal to 16% and in particular less than or equal to 10%.

9. Article according to any one of the preceding claims, **characterized in that** it has a storage modulus E' of greater than or equal to 1 MPa, especially ranging from 1 MPa to 5000 MPa, in particular greater than or equal to 5 MPa, especially ranging from 5 to 1000 MPa, and more particularly greater than or equal to 10 MPa, for example ranging from 10 to 500 MPa, at a temperature of 30°C and a frequency of 0.1 Hz.

10. Article according to any one of the preceding claims, **characterized in that** it has an ultimate strain εᵣ ranging from 5% to 500% and/or an energy at break per unit volume Wᵣ of greater than or equal to 0.2 J/cm³, especially ranging from 0.2 to 100 J/cm³.

11. Article according to any one of the preceding claims, **characterized in that** the crosslinking is performed thermally, photochemically and/or chemically, in the presence or absence of a catalyst.

12. Article according to any one of the preceding claims, **characterized in that** said crosslinking is of polyaddition and/or polycondensation type.

13. Article according to Claim 11 or 12,
**characterized in that** the crosslinked film is derived from the crosslinking of a reactive system formed by:
- at least one first compound (A) comprising at least two functions X,
- at least one second compound (B) comprising at least two functions Y that are reactive with the functions X.

14. Article according to Claim 13,
**characterized in that** said reactive system has a mean functionality (total number of functions X and Y/total number of molecules of compounds (A) and (B)) of greater than 2, in particular greater than or equal to 2.2 and especially ranging from 2.5 to 100.

15. Article according to Claim 13 or 14,
**characterized in that** said compound (A) and/or compound (B) is organic and is especially an oligomer, polymer or copolymer.

16. Article according to Claim 13, 14 or 15,
**characterized in that** said compound (A) and/or compound (B) is inorganic and bears at the surface said functions X or Y.

17. Article according to any one of Claims 13 to 16, **characterized in that** said functions X and Y are chosen from "reactive" functions and functions comprising at least one labile hydrogen.

18. Article according to any one of Claims 13 to 17, **characterized in that** the reactive functions are chosen from isocyanate, epoxide and ethylenic double bond functions.

19. Article according to Claim 17 or 18,
**characterized in that** the functions containing labile hydrogen(s) are of the carboxylic, alcohol, especially phenol, primary or secondary amine, amide, amino alcohol and/or thiol type.

20. Article according to either of Claims 12 and 13, **characterized in that** said crosslinking uses at least one first type of compound comprising reactive functions and a second type of compound comprising functions containing labile hydrogen(s).

21. Article according to Claim 20,
**characterized in that** the reactive functions are epoxide and/or isocyanate functions.

22. Article according to any one of Claims 18 to 21, **characterized in that** the compounds bearing reactive functions of isocyanate type are chosen from aliphatic, cycloaliphatic and aromatic diisocyanates, triisocyanates and polyisocyanates with a molecular mass of less than 10 000.

23. Article according to Claim 22,
**characterized in that** said compounds are chosen from:
- 1,4-tetramethylene diisocyanate, 1,6-hexamethylene diisocyanate, 2,6- and 2,4-toluene diisocyanate, diphenylmethane diisocyanate and isophorone diisocyanate,
- the triisocyanates of formulae: 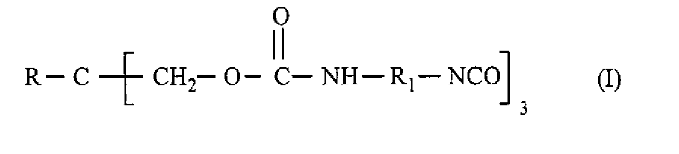 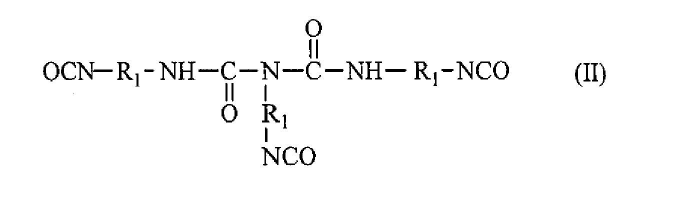 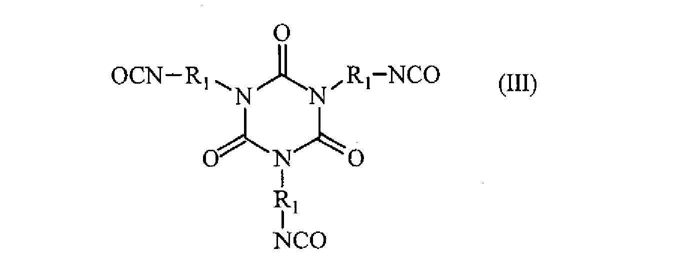 with R being an alkyl radical containing from 1 to 30 carbon atoms, and each R₁ independently representing a linear, branched or cyclic divalent hydrocarbon-based radical containing from 2 to 30 carbon atoms,
- polycondensates containing isocyanate end or side groups, such as polyurethanes, polyureas, polyesters, polyamides, polyepoxy, polyethers and/or perfluoropolyethers, and
- polymers resulting from the copolymerization of vinyl, allylic and/or (meth)acrylic monomers and of ethylenically unsaturated comonomers comprising a free isocyanate function.

24. Article according to any one of Claims 18 to 23, **characterized in that** the compounds bearing reactive functions of epoxide type are chosen from:
- bisphenol A diglycidyl ether,
- diepoxy resins,
- epoxy ester resins containing α,ω-diepoxy end groups,
- epoxy ether resins containing α,ω-diépoxy end groups,
- natural or synthetic oils bearing at least two epoxide groups,
- oligomers or polymers resulting from the copolymerization of unsaturated or vinyl, allylic and/or (meth)acrylic monomers, and of ethylenically unsaturated comonomers comprising a free epoxide function, and
- polycondensates containing epoxy end and/or side groups.

25. Article according to any one of Claims 18 to 24, **characterized in that** the compounds bearing functions containing labile hydrogen(s) are chosen from:
- aliphatic diols,
- polyols,
- aliphatic, cycloaliphatic or aromatic diamines,
- multifunctional amines containing more than two amine groups,
- oligomers bearing at least two amine groups,
- alkyd resins, and
- dendrimers or hyperbranched polymers whose chain end groups are primary amines.

26. Article according to any one of Claims 1 to 11, **characterized in that** said crosslinking is performed photochemically and involves at least two compounds (A) and (B) bearing functions of unsaturated double bond type in the presence of a photoinitiator.

27. Article according to Claim 26,
**characterized in that** (A) and (B) are chosen so as to form a reactive system whose mean valency is greater than 2.

28. Article according to Claim 27,
**characterized in that** compounds (A) and (B) are chosen from:
a) ethylenically unsaturated polyesters,
b) polyesters containing (meth)acrylate side and/or end groups,
c) polyurethanes and/or polyureas containing (meth)acrylate groups,
d) poly (C₁₋₅₀ alkyl) (meth) acrylates comprising at least two ethylenic double bond functions borne by the hydrocarbon-based side and/or end chains,
e) polyorganosiloxanes containing (meth)acrylate or (meth)acrylamide groups,
f) dendrimers and hyperbranched polymers bearing (meth)acrylate or (meth)acrylamide end groups, and
g) ethylenically unsaturated monomers.

29. Article according to any one of the preceding claims, **characterized in that** said film also comprises a catalyst.

30. Article according to any one of Claims 1 to 29, **characterized in that** it also comprises at least one dyestuff, especially pigments, nacres and/or flakes.

31. Article according to any one of the preceding claims, **characterized in that** it also comprises organic or inorganic solid particles.

32. Article according to any one of the preceding claims, **characterized in that** said adhesive layer comprises at least one adhesive material.

33. Article according to any one of the preceding claims, **characterized in that** said adhesive material is such that said article cannot be removed by peeling when it is applied to the surface of a synthetic or natural nail after an application time of at least 24 hours.

34. Article according to Claim 32 or 33,
**characterized in that** said adhesive material is chosen from copolymers derived from the copolymerization of vinyl monomers with polymeric species, copolymers bearing a polymer backbone, with a Tg ranging from 0°C to 45°C, grafted with chains derived from acrylic and/or methacrylic monomers and having, in contrast, a Tg ranging from 50°C to 200°C and polyisobutylenes with a relative molar mass Mv of greater than or equal to 10 000 and less than or equal to 150 000.

35. Article according to any one of the preceding claims, **characterized in that** said adhesive layer is layered at the surface with a removable support.

36. Article according to the preceding claim,
**characterized in that** said removable support consists of a plastic film modified by means of a surface treatment with silicone or with salts of C₁₂ to C₂₂ fatty acids.

37. Product for making up and/or caring for the nails and/or false nails, comprising, in substantially airtight packaging, at least one flexible article intended to be applied to the nails and/or false nails for making them up and/or caring for them, comprising:
- at least one adhesive layer for fixing the article to the nail, and
- at least one crosslinked film, which can be removed using a solvent, the said crosslinked film possibly being in accordance with any one of Claims 4 to 6 and 11 to 29 and the said article also possibly comprising the characteristics according to Claims 30 to 36, the packaging being such that the said article is conserved therein in a partially dry form.

38. Product according to Claim 37,
**characterized in that** said article has a solids content of less than 80%, in particular less than 75% and more particularly less than 70% by weight relative to the total weight of said article.

39. Product according to Claim 37 or 38,
**characterized in that** the packaging comprises a reservoir capable of containing said article in a leaktight manner.

40. Process for preparing a flexible article for making up and/or caring for the nails, comprising at least the steps consisting in superposing on a removable support:
a) at least one layer of a composition based on at least one adhesive material, and
b) at least one layer of a crosslinkable composition, the crosslinking of said composition being performed consecutively to its deposition so as to obtain a crosslinked film.

41. Process according to Claim 40,
**characterized in that** a film of colored varnish is also formed between the adhesive layer and the crosslinked film.

42. Process according to Claim 41,
**characterized in that** the film of varnish is obtained by crosslinking and/or evaporation of the organic or aqueous solvent phase of a solution or dispersion of at least one film-forming polymer.

43. Process according to any one of Claims 40 to 42, **characterized in that** the crosslinking is of polyaddition and/or polycondensation type as defined in Claims 12 to 25.

44. Process according to any one of Claims 40 to 42, **characterized in that** the crosslinking is performed photochemically as defined in Claims 25 to 27.

45. Process according to any one of Claims 40 to 44, **characterized in that** the adhesive material is as defined in Claim 33 or 34.

46. Process for preparing a product according to either of Claims 37 and 38, comprising the steps consisting in superposing on a removable support:
a) at least one layer of a composition based on at least one adhesive material,
b) at least one layer of a crosslinkable composition, the crosslinking of said film being performed consecutively to the deposition of said composition,
c) if necessary, partial drying of the article thus obtained, and
d) packaging of said article in a partially dry form in substantially airtight packaging.

47. Process for making up the nails using an article as defined in Claims 1 to 36, comprising the fact that the adhesive face of said article is applied to a natural or synthetic nail.

## Patentansprüche

1. Elastischer Gegenstand, der dazu ausgelegt ist, auf die Fingernägel oder auf künstliche Fingernägel zu ihrer Übermalung oder zu ihrer Pflege aufgebracht zu werden, umfassend:
- mindestens eine Klebeschicht, die die Fixierung des Gegenstands auf dem Nagel erlaubt und
- mindestens einen vernetzten Film,
**dadurch gekennzeichnet, dass** er einen Gehalt an Trockenmasse über 80 % besitzt, bezogen auf sein Gesamtgewicht, und **dadurch** dass er mit Hilfe eines Alkylacetat-Lösungsmittels abgeschminkt werden kann.

2. Elastischer Gegenstand, der dazu ausgelegt ist, auf die Fingernägel oder auf künstliche Fingernägel zu ihrer Übermalung oder zu ihrer Pflege aufgebracht zu werden, umfassend:
- mindestens eine Klebeschicht, die die Fixierung des Gegenstands auf dem Nagel erlaubt und
- mindestens einen vernetzten Film,
**dadurch gekennzeichnet, dass** er einen Gehalt an Trockenmasse über 80 % besitzt, bezogen auf sein Gesamtgewicht, und **dadurch** dass er eine Bruchverformung ε_{τ} von größer oder gleich 5 % aufweist.

3. Gegenstand nach Anspruch 1 oder 2, **dadurch gekennzeichnet, dass** er weiterhin zwischen der Klebeschicht und dem vernetzten Film mindestens einen Farblackfilm einschließt.

4. Gegenstand nach einem der Ansprüche 1 bis 3, **dadurch gekennzeichnet, dass** der vernetzte Film transparent ist.

5. Gegenstand nach Anspruch 3 oder 4, **dadurch gekennzeichnet, dass** der Lackfilm durch Vernetzung oder Verdampfung der organischen oder wässrigen Lösungsmittelphase von einer Lösung oder Dispersion aus mindestens einem filmbildenden Polymer erhalten wird.

6. Gegenstand nach Anspruch 5, **dadurch gekennzeichnet, dass** das filmbildende Polymer aus Nitrocellulose oder Celluloseestern ausgewählt ist.

7. Gegenstand nach einem der vorhergehenden Ansprüche, **dadurch gekennzeichnet, dass** er einen Gehalt an Trockenmasse von über 85 Gew.-%, bezogen auf sein Gesamtgewicht, aufweist.

8. Gegenstand nach einem der vorhergehenden Ansprüche, **dadurch gekennzeichnet, dass** er einen Wassersorptionsbereich bei 25 °C von kleiner oder gleich 20 %, insbesondere von kleiner oder gleich 16 % und speziell von kleiner oder gleich 10 % aufweist.

9. Gegenstand nach einem der vorhergehenden Ansprüche, **dadurch gekennzeichnet, dass** er einen Erhaltungsmodul E' von größer oder gleich 1 MPa, insbesondere im Bereich von 1 MPa bis 5000 MPa, speziell von größer oder gleich 5 MPa, insbesondere im Bereicht von 5 bis 1000 MPa, und ganz besonders von größer oder gleich 10 MPa, zum Beispiel im Bereich von 10 bis 500 MPa, bei einer Temperatur von 30 °C und einer Frequenz von 0,1 Hz aufweist.

10. Gegenstand nach einem der vorhergehenden Ansprüche, **dadurch gekennzeichnet, dass** er eine Bruchverformung ε_{τ} im Bereich von 5 bis 500 % und/oder eine Bruchenergie pro Volumeneinheit Wᵣ von größer oder gleich 0,2 J/cm³, insbesondere im Bereich von 0,2 bis 100 J/cm³ aufweist.

11. Gegenstand nach einem der vorhergehenden Ansprüche, **dadurch gekennzeichnet, dass** die Vernetzung thermisch, photochemisch und/oder chemisch in Gegenwart von einem oder von keinem Katalysator durchgerührt wird.

12. Gegenstand nach einem der vorhergehenden Ansprüche, **dadurch gekennzeichnet, dass** die Vernetzung vom Typ einer Polyaddition und/oder Polykondensation ist.

13. Gegenstand nach Anspruch 11 oder 12, **dadurch gekennzeichnet, dass** der vernetzte Film aus der Vernetzung eines reaktiven Systems stammt, das gebildet wird durch:
- mindestens eine erste Verbindung (A), die mindestens zwei Funktionen X umfasst,
- mindestens eine zweite Verbindung (B), die mindestens zwei Funktionen Y umfasst, die gegenüber den Funktionen X reaktiv sind.

14. Gegenstand nach Anspruch 13, **dadurch gekennzeichnet, dass** das reaktive System eine durchschnittliche Funktionalität (Gesamtanzahl der Funktionen X und Y/Gesamtanzahl der Moleküle von Verbindungen (A) und (B)) von größer als 2, speziell von größer oder gleich 2,2 und insbesondere von 2,5 bis 100 variierend aufweist.

15. Gegenstand nach Anspruch 13 oder 14, **dadurch gekennzeichnet, dass** die Verbindung
(A) und/oder die Verbindung (B) organisch ist und insbesondere ein Oligomer, Polymer oder ein Copolymer ist,

16. Gegenstand nach Anspruch 13, 14 oder 15, **dadurch gekennzeichnet, dass** die Verbindung (A) und/oder die Verbindung (B) anorganisch ist und an der Oberfläche die Funktionen Y und Y aufweist.

17. Gegenstand nach einem der Ansprüche 13 bis 16, **dadurch gekennzeichnet, dass** die Funktionen X und Y aus Funktionen, die reaktiv genannt werden, und aus Funktionen, die mindestens einen labilen Wasserstoff einschließen, ausgewählt sind.

18. Gegenstand nach einem der Ansprüche 13 bis 17, **dadurch gekennzeichnet, dass** die reaktiven Funktionen aus Isocyanatfunktionen, Epoxidfunktionen und ethylenischen Doppelbirrdungsfunktionen ausgewählt sind.

19. Gegenstand nach Anspruch 17 oder 18, **dadurch gekennzeichnet, dass** die Funktionen mit labilem(n) Wasserstoff(en) vom carboxylischen Typ, vom Typ eines Alkohols, insbesondere vom phenolischen Typ, von Typ eines primären oder sekundären Amins, Amids, Aminoalkohols und/oder Thiols sind.

20. Gegenstand nach einem der Ansprüche 12 bis 13, **dadurch gekennzeichnet, dass** die Vernetzung mindestens einen ersten Typ von Verbindung, der reaktive Funktionen einschließt, und einen zweiten Typ von Verbindung, der Funktionen mit labilem(n) Wasserstoff(en) einschließt, verwendet.

21. Gegenstand nach Anspruch 20, **dadurch gekennzeichnet, dass** die reaktiven Funktionen Epoxidfunktionen und/oder Isocyanatfunktionen sind.

22. Gegenstand nach einem der Ansprüche 18 bis 21, **dadurch gekennzeichnet, dass** die Verbindungen, die reaktive Funktionen von Typ eines Isocyanats tragen, aus Diisocyanaten, Triisocyanaten und aliphatischen und cycloaliphatischen oder aromatischen Polyisocyanaten mit einer Molekülmasse von kleiner als 10000 ausgewählt sind.

23. Gegenstand nach Anspruch 22, **dadurch gekennzeichnet, dass** die Verbindungen ausgewählt sind aus:
- 1,4-Tetramethylendiisocyanat, 1,6-Hexamethylendiisocyanat, 2,6- und 2,4-Toluoldiisocyanat, Diphenylmethandiisocyanat und Isophorondiisocyanat,
- Triisocyanaten der Formen: 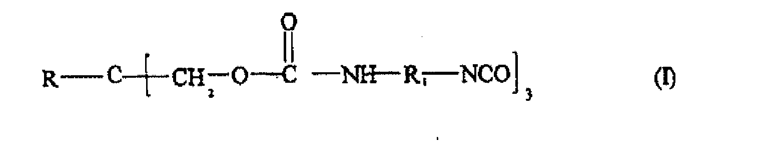 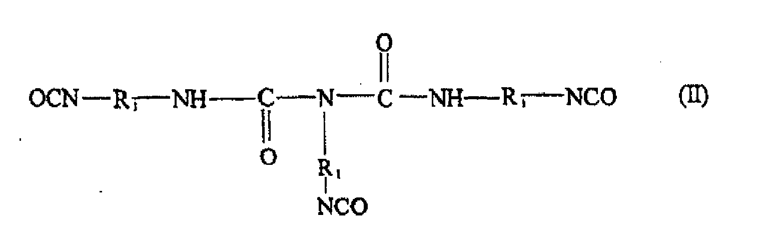 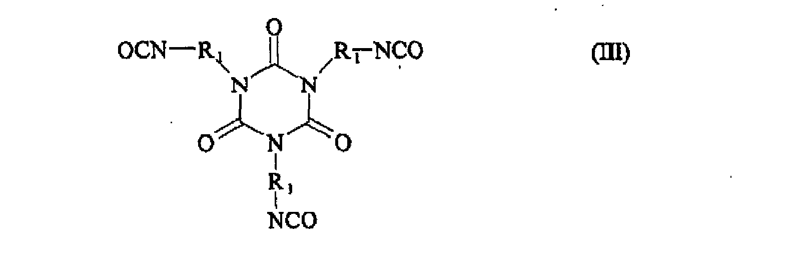
wobei R ein Alkylrest ist, der 1 bis 30 Kohlenstoffatome umfasst, und R₁ jeweils unabhängig eine zweiwertigen linearen, verzweigten oder cyclischen Kohlenwasserstoffrest mit 2 bis 30 Kohlenstoffatomen umfasst;
- Polykondensaten mit terminalen oder lateralen Isocyanatgruppen, wie Polyurethane, Polyharnstoffe, Polyester, Polyamide, Polyepoxy, Polyether und/oder Perfluorpolyether, und
- Polymeren, die aus der Copolymerisation von vinylischen, allylischen und/oder (meth)acrylischen Monomeren und Comonomeren mit ethylenischer Ungesättigtheit, die eine freie Isocyanatfunktion umfassen, hervorgehen.

24. Gegenstand nach einem der Ansprüche 18 bis 23, **dadurch gekennzeichnet, dass** die Verbindungen, die reaktive Funktionen von Typ eines Epoxids tragen, ausgewählt sind aus:
- Bisphenol A-diglycidylether,
- Diepoxyharzen,
- Epoxyesterharzen mit α,ω-Diepoxyenden,
- Epoxyetherharzen mit α,ω-Diepoxyenden,
- natürlichen oder synthestischen Ölen, die mindestens zwei Epoxidgruppen tragen,
- Oligimeren oder Polymeren, die aus der Copolymerisation von vinylischen, allylischen und/oder (meth)acrylischen Monomeren und Comonomeren mit ethylenischer Ungesättigtheit, die eine freie isocyanatfuraktion einschließen, hervorgehen, und
- Polykondensaten mit terminalen und/oder lateralen Epoxygruppen.

25. Gegenstand nach einem der Ansprüche 18 bis 24, **dadurch gekennzeichnet, dass** die Verbindungen, die Funktionen mit labilem(n) Wasserstoff(en) tragen, ausgewählt sind aus:
- aliphatischen Diolen,
- Polyolen,
- aliphatischen, cycloaliphatischen oder aromatischen Diaminen,
- multifunktionellen Aminen mit mehr als zwei Amingruppen,
- Oligomeren, die mindestens zwei Amingruppen tragen,
- Alkydharzen, und
- Dendrimeren oder sehr stark verzweigten Polymeren, deren Ketten-Enden primäre Amine sind.

26. Gegenstand nach einem der Ansprüche 1 bis 11, **dadurch gekennzeichnet, dass** die Vernetzung photochemisch in Gegenwart eines Photostarters durchgeführt wird und mindestens zwei Verbindungen (A) und (B), die Funktionen vom Typ ungesättigter Doppelbindungen besitzen, verwendet.

27. Gegenstand nach Anspruch 26, **dadurch gekennzeichnet, dass** (A) und (B) so gewühlt sind, dass sie ein reaktives System bilden, dessen durchschnittliche Valenz größer als 2 ist.

28. Gegenstand nach Anspruch 27, **dadurch gekennzeichnet, dass** die Verbindungen (A) und (B) ausgewählt sind aus:
a) Polyestern mit ethylenischer(n) Ungesättigtheit(en),
b) Polyestern mit lateralen und/oder terminalen (Meth)acrylatgruppen,
c) Polyurethanen und/oder Polyharnstoffen mit Meth)acrylatgruppen,
d) (C₁₋₅₀-Alkyl)poly(meth)acrylaten, die mindestens zwei Funktionen mit ethylenischer Doppelbindung umfassen, die von den lateralen und/oder terminalen Kohlenwasserstoffketten getragen werden,
e) Polyorganosiloxanen mit (Meth)acrylat- oder (Meth)acrylamidgruppen,
f) Dendrimeren und sehr stark verzweigten Polymeren, die terminale (Meth)acrylat- oder (Meth)acrylamidgruppen tragen, und
g) Monomeren mit ethylenischer Ungesättigtheit.

29. Gegenstand nach einem der vorhergehenden Ansprüche, **dadurch gekennzeichnet, dass** der Film weiterhin einen Katalysator umfasst.

30. Gegenstand nach einem der Ansprüche 1 bis 29, **dadurch gekennzeichnet, dass** er weiterhin mindestens einen Farbstoff, insbesondere Pigmente, Perlmuttlacke und Glitter einschließt.

31. Gegenstand nach einem der vorhergehenden Ansprüche, **dadurch gekennzeichnet, dass** er weiterhin organische oder anorganische feste Teilchen einschließt.

32. Gegenstand nach einem der vorhergehenden Ansprüche, **dadurch gekennzeichnet, dass** die Klebeschicht mindestens einen Klebstoff einschließt.

33. Gegenstand nach einem der vorhergehenden Ansprüche, **dadurch gekennzeichnet, dass** der Klebstoff so ist, dass der Gegenstand nicht durch Abziehen entfernt werden kann, wenn er auf die Oberfläche eines synthetischen oder natürlichen Nagels aufgebracht wird, mindestens 24 Stunden nach dem Auflegen.

34. Gegenstand nach Anspruch 32 oder 38, **dadurch gekennzeichnet, dass** der Klebstoff ausgewählt ist aus Copolymeren, die sich von der Copolymerisation von vinylischen Monomeren mit polymeren Einheiten, Copolymeren, die ein Polymerskelett besitzen, mit einer T_{g}, die von 0 °C bis 45 °C variiert, die mit Ketten gepfropft sind, die sich von acrylischen und/oder methacrylischen Monomeren ableiten und die im Gegenzug eine T_{g} besitzen, die von 50 °C bis 200 °C variiert, ableiten, und Polyisobutylenen, die eine relative Molmasse Mv von größer oder gleich 10000 und von kleiner oder gleich 150000 aufweisen.

35. Gegenstand nach einem der vorhergehenden Ansprüche, **dadurch gekennzeichnet, dass** die Klebeschicht auf die Oberfläche von einem ablösbaren Träger aufgebracht ist.

36. Gegenstand nach dem vorhergehenden Anspruch, **dadurch gekennzeichnet, dass** der ablösbaren Träger aus einem plastischen Film besteht, der durch eine OberflächenBehandlung durch Silikon oder durch C₁₂- bis C₂₂-Fettsäuresalze modifiziert wurde.

37. Produkt zum Überdecken und/oder zur Pflege der Fingernägel oder der künstlichen Nägel, umfassend in einem im Wesentlichen luftundurchlässigen Behälter mindestens einen elastischen Gegenstand, der dazu bestimmt ist, auf die Fingernägel oder auf künstliche Nägel zu ihrer Übermalung oder Pflege aufgebracht zu werden, umfassend:
- mindestens eine Klebeschicht, die die Fixierung des Gegenstands auf dem Nagel erlaubt und
- mindestens einen vernetzten Film,
das mit Hilfe eines Lösungsmittel abgeschminkt werden kann, wobei der vernetzte Film gemäß einem der Ansprüche 4 bis 6 und 11 bis 29 sein kann und der Gegenstand weiterhin die Merkmale gemäß einem der Ansprüche 30 bis 36 einschließen kann, wobei der Behälter so ist, dass sich der Gegenstand darin in einer teilweise trockenen Form konserviert befindet.

38. Produkt nach Anspruch 37, **dadurch gekennzeichnet, dass** der Gegenstand einen Gehalt an Trockenmasse von unter 80 %, insbesondere von unter 75 % und ganz besonders von unter 70 % besitzt, bezogen auf das Gesamtgewicht des Gegenstands.

39. Produkt nach Anspruch 37 oder 38, **dadurch gekennzeichnet, dass** der Behälter ein Reservoir umfasst, das dazu ausgelegt ist, den Gegenstand luftdicht zu enthalten.

40. Verfahren zur Herstellung eines elastischen Gegenstands zum Übermalen und/oder zur Pflege der Nägel, das mindestens die Schritte umfasst, die bestehen aus dem Übereinanderlegen auf einem ablösbaren Träger von:
a) mindestens einer Schicht aus einer Zusammensetzung auf der Grundlage von mindestens einem Klebstoff, und
b) mindestens einer Schicht aus einer vernetzbaren Zusammensetzung, wobei die Vernetzung der Zusammensetzung auf die Ablagerung von dieser folgend so durchgeführt wird, dass ein vernetzter Film erhalten wird.

41. Verfahren nach Anspruch 40, **dadurch gekennzeichnet, dass** weiterhin zwischen der Klebeschicht und dem vernetzten Film ein Film aus Farblack gebildet wird.

42. Verfahren nach Anspruch 41, **dadurch gekennzeichnet, dass** der Lackfilm durch Vernetzung und/oder Verdampfung der organischen oder wässrigen Lösungsmittelphase von einer Lösung oder Dispersion von mindestens einem filmbildenden Polymer erhalten wird.

43. Verfahren nach einem der Ansprüche 40 bis 42, **dadurch gekennzeichnet, dass** die Vernetzung vom Typ einer Polyaddition und/oder Polykondensation ist, wie in den Ansprüchen 12 bis 25 definiert.

44. Verfahren nach einem der Ansprüche 40 bis 42, **dadurch gekennzeichnet, dass** die Vernetzung photochemisch durchgeführt wird, wie in den Ansprüchen 25 bis 27 definiert.

45. Verfahren nach einem der Ansprüche 40 bis 44, **dadurch gekennzeichnet, dass** der Klebstoff so ist, wie in den Ansprüchen 33 oder 34 definiert.

46. Verfahren zur Herstellung eines Produkt nach einem der Ansprüche 37 bis 38, das die Schritte umfasst, die bestehen aus dem Übereinanderlegen auf einem ablösbaren Träger von:
a) mindestens einer Schicht aus einer Zusammensetzung auf der Grundlage von mindestens einem Klebstoff,
b) mindestens einer Schicht aus einer vernetzbaren Zusammensetzung, wobei die Vernetzung des Films auf die Ablagerung der Zusammensetzung folgend durchgeführt wird,
c) sofern notwendig, teilweises Trocknen des so erhaltenen Gegenstands, und
d) die Verpackung des Gegenstands in einem teilweise getrockneten Zustand in einem im Wesentlichen luftdichten Behälter.

47. Verfahren zum Übermalen der Nägel unter Verwendung eines Gegenstands wie in Anspruch 1 bis 36 definiert, umfassend das Aufbringen der Klebefläche des Gegenstands auf einen natürlichen oder synthetischen Nagel.
